# EUROPEAN PATENT SPECIFICATION

(11) **EP 3 643 792 B1**
(45) Date of publication and mention of the grant of the patent: **15.09.2021**
(21) Application number: 18202404.2
(22) Date of filing: 24.10.2018
(51) Int. Cl.: C12Q 1/6837

(54) **DETECTION AND ANALYSIS OF SEQUENCE VARIATIONS IN A TARGET NUCLEIC ACID**
NACHWEIS UND ANALYSE VON SEQUENZVARIATIONEN IN EINER ZIEL-NUKLEINSÄURE
DÉTECTION ET ANALYSE DE VARIATIONS DE SÉQUENCE DANS UN ACIDE NUCLÉIQUE CIBLE

(43) Date of publication of application: 29.04.2020
(73) Proprietor: Fujirebio Europe N.V., 9052 Gent (BE)
(72) Inventor: Mersch, Guy, 1700 Dilbeek (BE); Troch, Kurt, 9120 Melsele (BE); Jannes, Geert, 3010 Kessel Lo (BE)
(74) Representative: De Clercq & Partners

(56) References cited:
- WO-A2-94/12670
- E GEORGE; ET AL: "Beta Thalassaemia Mutations in Malays: a simplified cost-effective strategy to identify the mutations", MALAYSIAN JOURNAL OF MEDICINE AND HEALTH SCIENCES, vol. 8, 1 January 2012 (2012-01-01), XP055574093,
- Anonymous ET AL: "INNO-LiPA CFTR19: Instruction manual", , 11 December 2017 (2017-12-11), XP055574117, ISBN: 978-1-339-60234-9 Retrieved from the Internet: URL:https://www.e-labeling.eu/FRI54885/498 31/EN [retrieved on 2019-03-25]
- A. E. KRAFFT ET AL: "Time-Motion Analysis of 6 Cystic Fibrosis Mutation Detection Systems", CLINICAL CHEMISTRY, vol. 51, no. 7, 1 July 2005 (2005-07-01), pages 1116-1122, XP055574066, ISSN: 0009-9147, DOI: 10.1373/clinchem.2004.047423
- MARCO LUCARELLI ET AL: "A New Targeted CFTR Mutation Panel Based on Next-Generation Sequencing Technology", THE JOURNAL OF MOLECULAR DIAGNOSTICS, vol. 19, no. 5, 1 September 2017 (2017-09-01), pages 788-800, XP055574103, US ISSN: 1525-1578, DOI: 10.1016/j.jmoldx.2017.06.002

## Description

### FIELD OF THE INVENTION

The invention relates to the field of molecular diagnostics. More particularly methods and tools are provided for the analysis of sequence variations in a target nucleic acid e.g. for the diagnosis of multimutational diseases or for the genetic classification of microorganisms.

### BACKGROUND OF THE INVENTION

For certain genes, the sequence variation in a population can be enormous. For example, nearly 2000 putative cystic fibrosis (CF)-associated mutations have been reported across the cystic fibrosis transmembrane regulator (*CFTR*) gene, some though with a very low frequency in the general population, which makes the detection of cystic fibrosis complex. The identification of carriers of *CFTR* mutations is even more complex, further requiring the detection of the corresponding wild-type sequences. Thus, efficient and accurate population screening for cystic fibrosis requires an assay that can simultaneously detect multiple mutant and wild-type sequences of the *CFTR* gene.

Other diseases such as cancers are associated with multiple mutations in several genes, requiring the simultaneous detection of sequence variations in multiple genes.

Genetic variability also exists in microbial genomes; with certain genotypes causing infections in animals or humans while others not and/or some genotypes being associated with resistance to certain therapies. The genotyping and/or genetic classification of microorganisms also require the simultaneous detection of multiple, wild-type and mutant, sequences in one or more genes of the microbial genome.

A variety of methods exist for identifying variations in a target nucleic acid sequence. Exemplary assays are reverse-hybridization assays, wherein specific oligonucleotide probes are immobilized to a known location on a support and to which amplification products of the target nucleic acid are hybridized to detect the presence or absence of a particular target sequence. Contrary to cumbersome and more expensive sequencing-based approaches, reverse-hybridization assays, such as line probe (LiPA) assays (Stuyver et al. 1993; WO 94/12670), are straightforward and easy to perform, not requiring expensive instrumentation. The LiPA assay is particularly advantageous since it is fast and simple to perform. Moreover, this assay is amenable to automation (Auto-LiPA, Fujirebio Europe, Ghent, Belgium) and thus particularly suitable for clinical settings where multiple samples can be processed simultaneously. However, they are limited in the number of oligonucleotides that can be immobilized on a support and hence on the sequence variation that can simultaneously be analysed. In some cases, e.g. to ensure sufficient coverage of the *CFTR* mutations, multiple assays need to be performed to complete the genetic analysis, which is time-consuming and requires more reagents, thereby increasing the cost of the assay.

Accordingly, there is a need for efficient and simple methods that allow the simultaneous analysis of multiple sequence variations in a target nucleic acid. More particularly, there is need to improve current reverse-hybridization-based approaches, more particularly to increase the sequence variation that can be analysed without having to increase the assays to be performed.

### SUMMARY OF THE INVENTION

The present invention solves one or more of the above-mentioned problems in the art. More particularly, methods and tools are provided herein for the analysis of sequence variations in a target nucleic acid based on reverse-hybridization assays, wherein in a first hybridization reaction relevant amplification products of the target nucleic acid are hybridized with pools of oligonucleotide probes, followed by a second hybridization reaction with the individual oligonucleotides probes that are comprised in the positive pools. The positive pools in the first hybridization thus indicate which reverse-hybridization assays with individual oligonucleotide probes need to be performed in the second hybridization to complete the genetic analysis of the target nucleic acid. Advantageously, this methodology allows to reduce the number of reverse-hybridization assays with individual oligonucleotide probes that need to be performed to complete the genetic analysis.

The present invention is in particular captured by any one or any combination of one or more of the below numbered aspects and embodiments (i) to (xvi) wherein:
(i) A method for detection of multiple target sequences from a target nucleic acid and/or genetic analysis of a target nucleic acid in a sample, comprising:
   (a) optionally releasing, isolating or concentrating the nucleic acids present in the sample;
   (b) amplifying one or more regions of the target nucleic acid present in the sample with one or more suitable primer pairs, said one or more regions of the target nucleic acid comprising the target sequences to which the oligonucleotide probes hybridize;
   (c) performing a first hybridization reaction with the amplification products obtained in step (b) and a combination of at least two pools of oligonucleotide probes, wherein the oligonucleotide probes specifically hybridize to a target sequence from the target nucleic acid, wherein the oligonucleotide probes are capable of hybridizing to the amplification products under the same hybridization and wash conditions, and wherein said pools of oligonucleotide probes are immobilized to a known location on a first support;
   (d) detecting the hybrids formed in step (c);
   (e) performing a second hybridization reaction with the relevant amplification products obtained in step (b) and a combination of the oligonucleotide probes that are present in the pool(s) of oligonucleotide probes for which a positive hybridization signal was detected in step (d), wherein said oligonucleotide probes are individually immobilized to a known location on one or more second support(s);
   (f) detecting the hybrids formed in step (e); and
   (g) inferring the presence of at least one of the multiple target sequences from the target nucleic acid in the sample and/or the genotype of the target nucleic acid present in the sample from the differential hybridization signal(s) obtained in step (f).
(ii) The method according to embodiment (i), wherein in step (c) the amplification products are hybridized with a combination of at least three, four, five or more pools of oligonucleotide probes.
(iii) The method according to embodiment (i) or (ii), wherein the support is a solid support, preferably a membrane strip.
(iv) The method according to embodiment (iii), wherein the pools of oligonucleotide probes that are immobilized on the first support and the oligonucleotides probes that are individually immobilized on the one or more second support(s), are immobilized on a membrane strip in a line fashion.
(v) The method according to any one of embodiments (i) to (iv), wherein the target sequence is a mutant target sequence, a polymorphic target sequence or a wild-type target sequence.
(vi) The method according to any one of embodiments (i) to (v), wherein the first support further comprises individual oligonucleotide probes that specifically hybridize to a target sequence from the target nucleic acid, wherein said individual oligonucleotide probes are individually applied to a known location on said first support.
(vii) The method according to any one of embodiments (i) to (vi), wherein said first and second supports further comprise positive control oligonucleotide probes immobilized to a known location on said supports, wherein said positive control oligonucleotide probes hybridize to the amplification products obtained in step (b).
(viii) The method according to any one of embodiments (i) to (vii), wherein the target nucleic acid is genomic DNA.
(ix) The method according to embodiment (viii), wherein the target sequences are located within multiple genes present in said genomic DNA.
(x) The method according to embodiment (viii), wherein the target sequences are located within multiple regions of a gene present in said genomic DNA.
(xi) The method according to embodiment (x), wherein the gene is the cystic fibrosis transmembrane regulator (*CFTR*) gene.
(xii) The method according to embodiment (xi), wherein the oligonucleotide probes in the pools of oligonucleotide probes specifically hybridize to mutant target sequences from the *CFTR* gene.
(xiii) The method according to embodiment (xii), wherein in step (c) the amplification products are hybridized to a combination of pools of oligonucleotide probes comprising:
   a pool of oligonucleotide probes that permit detection of 2 or more, preferably all, of the *CFTR* mutations 3199del6, 711+1G>T, I507del, S1251N, R560T and R553X, wherein said oligonucleotide probes are for instance SEQ ID NO:1, SEQ ID NO:2, SEQ ID NO:3, SEQ ID NO:4, SEQ ID NO:5 and SEQ ID NO:6;
   a pool of oligonucleotide probes that permit detection of 2 or more, preferably all, of the *CFTR* mutations 3120+1G>A, Q552X, 3905insT, 3272-26A>G and 1898+1G>A, wherein said oligonucleotide probes are for instance SEQ ID NO:7, SEQ ID NO:8, SEQ ID NO:9, SEQ ID NO:10 and SEQ ID NO:11;
   a pool of oligonucleotide probes that permit detection of 2 or more, preferably all, of the *CFTR* mutations 2183AA>G, 2184delA, 394delTT, G85E, 2789+5G>A, R1162X, R117H and R347P, wherein said oligonucleotide probes are for instance SEQ ID NO:12, SEQ ID NO:13, SEQ ID NO:14, SEQ ID NO:15, SEQ ID NO:16, SEQ ID NO:17, SEQ ID NO:18 and SEQ ID NO:19; a pool of oligonucleotide probes that permit detection of 2 or more, preferably all, of the *CFTR* mutations R334W, 1078delT, 3849+10kbC>T, 3659delC, A455E, 2143delT, E60X and 711+5G>A, wherein said oligonucleotide probes are for instance SEQ ID NO:20, SEQ ID NO:21, SEQ ID NO:22, SEQ ID NO:23, SEQ ID NO:24, SEQ ID NO:25, SEQ ID NO:26 and SEQ ID NO:27;
   a pool of oligonucleotide probes that permit detection of 2 or more, preferably all, of the *CFTR* mutations 1259insA, 4016insT, 4382delA, E217G, D579G, G1244E, G1349D, I502T, L1065P, R1070Q and R334Q, wherein said oligonucleotide probes are for instance SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37 and SEQ ID NO:38;
   a pool of oligonucleotide probes that permit detection of 2 or more, preferably all, of the *CFTR* mutations R1158W, S549R(A\C), 991del5, D1152H, 1898+3A\G, R1066H, T338I, R347H, 621+3A>G and 852del22, wherein said oligonucleotide probes are for instance SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47 and SEQ ID NO:48;
   a pool of oligonucleotide probes that permit detection of 2 or more, preferably all, of the *CFTR* mutations dele1, dele2(ins182), dele22-24, dele14b-17b, 1706del17, R1066C and S912X, wherein said oligonucleotide probes are for instance SEQ ID NO:49, SEQ ID NO:50, SEQ ID NO:51, SEQ ID NO:52, SEQ ID NO:53, SEQ ID NO:54 and SEQ ID NO:55;
   a pool of oligonucleotide probes that permit detection of 2 or more, preferably all, of the *CFTR* mutations dele17a-17b-18, E585X, L1077P, dele22,23, dele2 and 1677delTA, wherein said oligonucleotide probes are for instance SEQ ID NO:56, SEQ ID NO:57, SEQ ID NO:58, SEQ ID NO:59, SEQ ID NO:60 and SEQ ID NO:61;
   a pool of oligonucleotide probes that permit detection of 2 or more, preferably all, of the *CFTR* mutations M1V, E92X, 1584+18672A>G, G178R, E822X, L206W, I336K, R352Q and M1101K, wherein said oligonucleotide probes are for instance SEQ ID NO:62, SEQ ID NO:63, SEQ ID NO:64, SEQ ID NO:65, SEQ ID NO:66, SEQ ID NO:67, SEQ ID NO:68, SEQ ID NO:69 and SEQ ID NO:70; and
   a pool of oligonucleotide probes that permit detection of 2 or more, preferably all, of the *CFTR* mutations 1609delCA, D110E, 1811+1,6kbA>G, 2184insA, 712-1G>T, Q890X, Y1092X, L346P and 4374+1G>A, wherein said oligonucleotide probes are for instance SEQ ID NO:71, SEQ ID NO:72, SEQ ID NO:73, SEQ ID NO:74, SEQ ID NO:75, SEQ ID NO:76, SEQ ID NO:77, SEQ ID NO:78 and SEQ ID NO:79.
(xiv) A set of arrays for detection of multiple target sequences from a target nucleic acid and/or genetic analysis of a target nucleic acid according to any one of methods (i) to (xiii), comprising:
   - a first array comprising a first support and at least two pools of oligonucleotide probes immobilized to a known location on said first support; and
   - at least two arrays comprising a second support and the oligonucleotide probes that are present in a pool of oligonucleotide probes of the first array individually immobilized to a known location on said second support,
   wherein the oligonucleotide probes specifically hybridize to a target sequence from the target nucleic acid, and wherein the oligonucleotide probes are capable of hybridizing to the amplification products under the same hybridization and wash conditions.
(xv) A kit for detection of multiple target sequences from a target nucleic acid and/or genetic analysis of a target nucleic acid according to any one methods (i) to (xiii), comprising:
   - one or more suitable primer pairs for amplifying the target nucleic acid region()s comprising the target sequences to which the oligonucleotide probes hybridize;
   - a set of arrays according to aspect (xiv);
   - a hybridization buffer, or components necessary for producing said buffer;
   - a wash solution, or components necessary for producing said wash solution;
   - optionally means for detection of the hybrids.
(xvi) A support for detection of multiple mutant target sequences from a CFTR gene, said support comprising pools of oligonucleotide probes immobilized to a known location on said support, wherein said pools of oligonucleotide probes comprise:
   a pool of oligonucleotide probes that permit detection of 2 or more, preferably all, of the *CFTR* mutations 3199del6, 711+1G>T, I507del, S1251N, R560T and R553X, wherein said oligonucleotide probes are for instance SEQ ID NO:1, SEQ ID NO:2, SEQ ID NO:3, SEQ ID NO:4, SEQ ID NO:5 and SEQ ID NO:6;
   a pool of oligonucleotide probes that permit detection of 2 or more, preferably all, of the *CFTR* mutations 3120+1G>A, Q552X, 3905insT, 3272-26A>G and 1898+1G>A, wherein said oligonucleotide probes are for instance SEQ ID NO:7, SEQ ID NO:8, SEQ ID NO:9, SEQ ID NO:10 and SEQ ID NO:11;
   a pool of oligonucleotide probes that permit detection of 2 or more, preferably all, of the *CFTR* mutations 2183AA>G, 2184delA, 394delTT, G85E, 2789+5G>A, R1162X, R117H and R347P, wherein said oligonucleotide probes are for instance SEQ ID NO:12, SEQ ID NO:13, SEQ ID NO:14, SEQ ID NO:15, SEQ ID NO:16, SEQ ID NO:17, SEQ ID NO:18 and SEQ ID NO:19; a pool of oligonucleotide probes that permit detection of 2 or more, preferably all, of the *CFTR* mutations R334W, 1078delT, 3849+10kbC>T, 3659delC, A455E, 2143delT, E60X and 711+5G>A, wherein said oligonucleotide probes are for instance SEQ ID NO:20, SEQ ID NO:21, SEQ ID NO:22, SEQ ID NO:23, SEQ ID NO:24, SEQ ID NO:25, SEQ ID NO:26 and SEQ ID NO:27;
   a pool of oligonucleotide probes that permit detection of 2 or more, preferably all, of the CFTR mutations 1259insA, 4016insT, 4382delA, E217G, D579G, G1244E, G1349D, I502T, L1065P, R1070Q and R334Q, wherein said oligonucleotide probes are for instance SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37 and SEQ ID NO:38;
   a pool of oligonucleotide probes that permit detection of 2 or more, preferably all, of the *CFTR* mutations R1158W, S549R(A\C), 991del5, D1152H, 1898+3A\G, R1066H, T338I, R347H, 621+3A>G and 852del22, wherein said oligonucleotide probes are for instance SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47 and SEQ ID NO:48;
   a pool of oligonucleotide probes that permit detection of 2 or more, preferably all, of the *CFTR* mutations dele1, dele2(ins182), dele22-24, dele14b-17b, 1706del17, R1066C and S912X, wherein said oligonucleotide probes are for instance SEQ ID NO:49, SEQ ID NO:50, SEQ ID NO:51, SEQ ID NO:52, SEQ ID NO:53, SEQ ID NO:54 and SEQ ID NO:55;
   a pool of oligonucleotide probes that permit detection of 2 or more, preferably all, of the *CFTR* mutations dele17a-17b-18, E585X, L1077P, dele22,23, dele2 and 1677delTA, wherein said oligonucleotide probes are for instance SEQ ID NO:56, SEQ ID NO:57, SEQ ID NO:58, SEQ ID NO:59, SEQ ID NO:60 and SEQ ID NO:61;
   a pool of oligonucleotide probes that permit detection of 2 or more, preferably all, of the *CFTR* mutations M1V, E92X, 1584+18672A>G, G178R, E822X, L206W, I336K, R352Q and M1101K, wherein said oligonucleotide probes are for instance SEQ ID NO:62, SEQ ID NO:63, SEQ ID NO:64, SEQ ID NO:65, SEQ ID NO:66, SEQ ID NO:67, SEQ ID NO:68, SEQ ID NO:69 and SEQ ID NO:70; and
   a pool of oligonucleotide probes that permit detection of 2 or more, preferably all, of the *CFTR* mutations 1609delCA, D110E, 1811+1,6kbA>G, 2184insA, 712-1G>T, Q890X, Y1092X, L346P and 4374+1G>A, wherein said oligonucleotide probes are for instance SEQ ID NO:71, SEQ ID NO:72, SEQ ID NO:73, SEQ ID NO:74, SEQ ID NO:75, SEQ ID NO:76, SEQ ID NO:77, SEQ ID NO:78 and SEQ ID NO:79.

### BRIEF DESCRIPTION OF THE FIGURES

The teaching of the application is illustrated by the following Figures which are to be considered as illustrative only and do not in any way limit the scope of the claims.

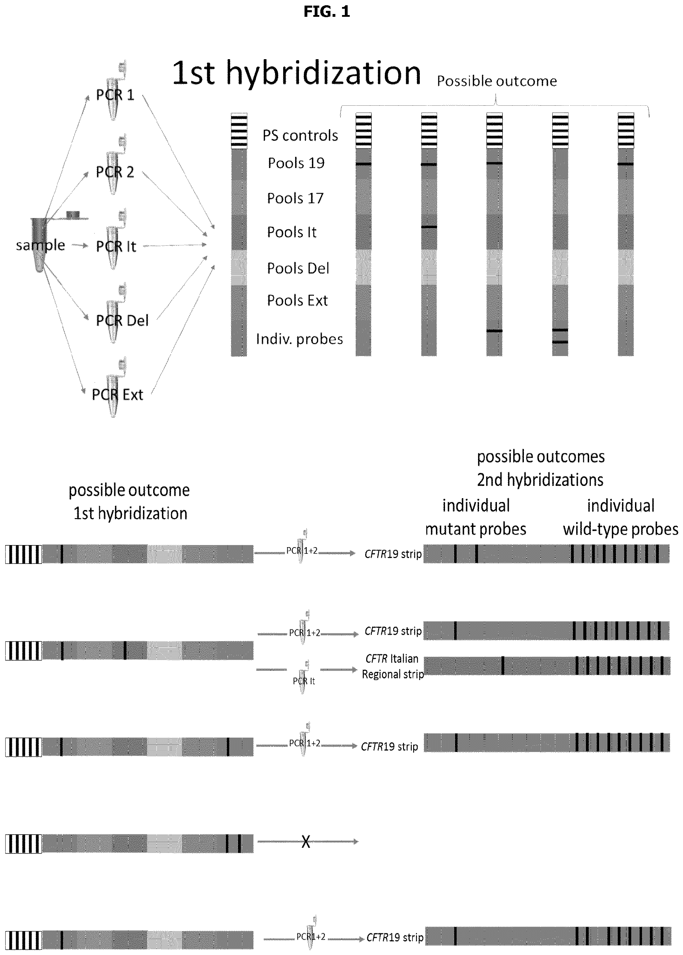

**Figure 1****:** Schematic illustration of a reverse-hybridization-based methodology for the analysis of sequence variations in a target nucleic acid according to an embodiment of the invention. "PCR 1", "PCR 2", "PCT It", "PCR Del" and "PCR Ext" refer to the amplification of *CFTR* gene regions by multiplex PCRs using respectively, the INNO-LiPA® Amplification *CFTR* kit (PCR 1 and PCR 2) and the reagents in the INNO-LiPA® CFTR tests CFTR Italian Regional, CFTR Deletions+6 and CFTR Extra (Fujirebio Europe, Ghent, Belgium). "Pools 19", "Pools 17", "Pools It", "Pools Del" and "Pools Ext" refer to the lines on the strip that comprise pools of oligonucleotide probes from respectively, the INNO-LiPA® CFTR strips CFTR19, CFTR17+Tn, CFTR Italian Regional, CFTR Deletions+6 and CFTR Extra (Fujirebio Europe, Ghent, Belgium). A detailed description of the strip and the composition of the pools is provided in Tables 2 and 1, respectively.
**Figure 2****:** Outcome for the sample L10016 on **(A)** the CFTRiage strip as described herein and **(B)** the INNO-LiPA® CFTR Italian Regional strip (Fujirebio Europe, Ghent, Belgium).
**Figure 3****:** Outcome for the sample L12711 on the *CFTR*iage strip as described herein.
**Figure 4****:** Outcome for the sample NA11496 on the *CFTR*iage strip as described herein.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention. When specific terms are defined in connection with a particular aspect of the invention or a particular embodiment of the invention, such connotation is meant to apply throughout this specification, i.e., also in the context of other aspects or embodiments of the invention, unless otherwise defined.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.
Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

The present application relates to methods and tools for the analysis of sequence variations in a target nucleic acid based on reverse-hybridization assays. More particularly, a method is provided herein for detection of multiple target sequences from a target nucleic acid and/or genetic analysis of a target nucleic acid in a sample, comprising:
(a) optionally releasing, isolating or concentrating the nucleic acids present in the sample;
(b) amplifying one or more regions of the target nucleic acid present in the sample with one or more suitable primer pairs, said one or more regions of the target nucleic acid comprising the target sequences to which the oligonucleotide probes hybridize;
(c) performing a first hybridization reaction with the amplification products obtained in step (b) and a combination of at least two pools of oligonucleotide probes, wherein the oligonucleotide probes specifically hybridize to a target sequence from the target nucleic acid, wherein the oligonucleotide probes are capable of hybridizing to the amplification products under the same hybridization and wash conditions, and wherein said pools of oligonucleotide probes are immobilized to a known location on a first support;
(d) detecting the hybrids formed in step (c);
(e) performing a second hybridization reaction with the relevant amplification products obtained in step (b) and a combination of the oligonucleotide probes that are present in the pool(s) of oligonucleotide probes for which a positive hybridization signal was detected in step (d), wherein said oligonucleotide probes are individually immobilized to a known location on one or more second support(s);
(f) detecting the hybrids formed in step (e); and
(g) inferring the presence of at least one of the multiple target sequences from the target nucleic acid in the sample and/or the genotype of the target nucleic acid present in the sample from the differential hybridization signal(s) obtained in step (f).

The term "genetic analysis" as used herein refers to the analysis of the nucleotide sequence of the target nucleic acid.

The target sequences can be any nucleotide sequence appearing in a target nucleic acid and may be a wild-type, polymorphic or mutated sequence.

As used herein, a "wild-type" target sequence refers to the standard or reference nucleotide sequence of the target nucleic acid to which variations are compared.

As used herein, a "polymorphic" target sequence refers to a naturally occurring variation in the nucleotide sequence of the target nucleic acid with usually a neutral or beneficial effect and which is common in a population (e.g. occurring at a frequency of 1% or higher).

A "mutant" or "mutated" target sequence as used herein refers to a change in the nucleotide sequence from the wild-type sequence that occurs in general less frequent in a population compared to a polymorphism and usually with a disease causing attribute.

The polymorphism and mutation can be a substitution, an addition (i.e. insertion), a deletion or a rearrangement, including a duplication, of one or more nucleotides.

The method described herein involves a reverse-hybridization approach, in particular a strip-based reverse-hybridization as described by Stuyver et al. (1993), which requires the design and/or assembly of a set of oligonucleotide probes that detect all relevant target sequences in one or more regions of the target nucleic acid.

The term "probe" refers to single stranded sequence-specific oligonucleotides that have a sequence which is complementary to the target sequence to be detected. Preferably, the target sequence is completely complementary to the sequence of the probe. The term "complementary" as used herein means that the oligonucleotide probe and the single-stranded target nucleic acid can form a base-paired double helix with each other. The sequence of the oligonucleotide probe may be the (inverse) complement of the sequence of the single-stranded target nucleic acid or a sequence wherein T of said target sequence is replaced by U (for RNA probes).

Since the method described herein requires the detection of single base-pair mismatches, stringent conditions for hybridization are required, allowing in principle only hybridization of exactly complementary sequences. It should be noted however that, since the central part of the probe is essential for its hybridization characteristics, possible deviations of the probe sequence versus the target sequence may be allowable towards head and tail of the probe when longer probe sequences are used. These variations, which may be conceived from the common knowledge in the art, should however always be evaluated experimentally, in order to check if they result in equivalent hybridization characteristics as the exactly complementary probes.

The oligonucleotide probes for use in a method described herein have as a common characteristic that they can be used together in a reverse-hybridization assay, more particularly under similar or identical hybridization and wash conditions as described elsewhere herein. Evidently, when other hybridization conditions would be preferred, all probes should be adapted accordingly by adding or deleting a number of nucleotides at their extremities. It should be understood that these concomitant adaptations should give rise to essentially the same result, namely that the respective probes still hybridize specifically with the defined target sequence. Such adaptations might also be necessary if the amplification products were RNA in nature and not DNA as in the case for the NASBA system.

For designing probes with desired characteristics, the following useful guidelines known to the person skilled in the art can be applied.

The stability of the [probe:target] nucleic acid hybrid should be chosen to be compatible with the assay conditions. This may be accomplished by avoiding long AT-rich sequences, by terminating the hybrids with G:C base pairs, and/or by designing the probe with an appropriate melting temperature (Tm). Preferably, the beginning and end points of the probe are chosen so that the length and %GC result in a Tm about 2-10°C higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be stable at higher temperatures.

Hybridization conditions such as ionic strength and incubation temperature under which a probe will be used should also be taken into account when designing a probe. It is known that hybridization will increase as the ionic strength of the reaction mixture increases, and that the thermal stability of the hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tm. In general, optimal hybridization for synthetic oligonucleotide probes of about 10-50 bases in length occurs approximately 5°C below the melting temperature for a given duplex. Incubation at temperatures below the optimum may allow mismatched base sequences to hybridize and can therefore result in reduced specificity.

In the present method, single base pair changes need to be detected, which requires conditions of very high stringency. Therefore, it is desirable to use probes in the method described herein which hybridize only under conditions of high stringency. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. The degree of stringency is chosen such as to maximize the difference in stability between the hybrid formed with the target and the non-target nucleic acid. The length of the target sequence and, accordingly, the length of the probe sequence can also be important. While it is possible for nucleic acids that are not perfectly complementary to hybridize, the longest stretch of perfectly complementary base sequence will normally primarily determine hybrid stability. In some cases, there may be several sequences from a particular region of a target nucleic acid, varying in location and length, which will yield probes with the desired hybridization characteristics. In other cases, one sequence may be significantly better than another which differs merely by a single base.

Regions in the target DNA or RNA which are known to form strong internal structures inhibitory to hybridization are less preferred. Likewise, probes with extensive self-complementarity should be avoided. As explained above, hybridization is the association of two single strands of complementary nucleic acids to form a hydrogen-bonded double strand. It is implicit that if one of the two strands is wholly or partially involved in a hybrid that it will be less able to participate in formation of a new hybrid. There can be intramolecular and intermolecular hybrids formed within the molecules of one type of probe if there is sufficient self-complementarity. Such structures can be avoided through careful probe design. By designing a probe so that a substantial portion of the target sequence is single stranded, the rate and extent of hybridization may be greatly increased. Computer programs are available to search for this type of interaction. However, in certain instances, it may not be possible to avoid this type of interaction.

Preferably, the probes are about 5 to 50 nucleotides long, more preferably from about 10 to about 25 nucleotides. Particularly preferred lengths of probes include 10, 11, 12, 13, 14, 15, 16, 17, 18, 19, 20, 21, 22, 23, 24 or 25 nucleotides. The nucleotides may be ribonucleotides, deoxyribonucleotides and modified nucleotides as described elsewhere herein or nucleotides containing modified groups which do not essentially alter their hybridisation characteristics.

Probe sequences are represented throughout the specification as single stranded DNA oligonucleotides from the 5' to the 3' end. It is obvious to the man skilled in the art that any specified probes disclosed herein can be used as such, or in their complementary form, or in their RNA form (wherein T is replaced by U).

The probes described herein can be prepared by cloning of recombinant plasmids containing inserts including the corresponding nucleotide sequences, if need be by cleaving the latter out from the cloned plasmids upon using the adequate nucleases and recovering them, e.g. by fractionation according to molecular weight. The probes can also be synthesized chemically, for instance by the conventional phospho-triester method.

Standard hybridization and wash conditions may be used in the method described herein, preferably stringent hybridization conditions are used, i.e. conditions enabling differentiation by hybridization between nucleic acids which differ by only one single nucleotide. Examples of stringent conditions are a hybridization buffer of 2xSSC (Sodium Saline Citrate) and 0.1% SDS at a hybridization temperature of 50° C. Other solutions (SSPE (Sodium saline phosphate EDTA), TMACI (Tetramethyl ammonium Chloride), etc.) and temperatures can also be used provided that the specificity and sensitivity of the probes is maintained. If need be, slight modifications of the probes in length or in sequence have to be carried out to maintain the specificity and sensitivity required under the given circumstances.

The reverse-hybridization approach implies that the oligonucleotide probes are immobilized to certain locations on a solid support and that the target nucleic acid is labelled in order to enable the detection of the hybrids formed.

The target nucleic acid may either be DNA or RNA, e.g. genomic DNA or messenger RNA or microbial DNA or RNA, including viral DNA or RNA, bacterial DNA or RNA and fungal DNA or RNA, or amplified versions thereof.

For use in a method described herein, the target nucleic acid may be obtained from any suitable "sample". Non-limiting examples of suitable samples include biological samples and environmental samples. Biological or clinical samples may be e.g. a sample taken from blood, from the respiratory tract (sputum, bronchoalveolar lavage (BAL)), from cerebrospinal fluid (CSF), from the urogenital tract (vaginal secretions, urine, sperm), from the gastrointestinal tract (saliva, faeces) or biopsies taken from organs, tissue, or skin from a human or animal, e.g. a human or animal suspected to be diagnosed with a certain disease or a human or animal suspected to be infected with a pathogen. The biological sample can be taken directly from the human or animal, or be obtained after culturing (enrichment). Non-limiting examples of environmental samples include surface matter, soil, water, and industrial samples.

Well-known extraction and purification procedures are available for the isolation of RNA or DNA from a sample (e.g. in Maniatis et al., Molecular Cloning: A Laboratory Manual, 2nd Edition, Cold Spring Harbour Laboratory Press (1989)) and a multitude of commercial kits for extraction and purification of RNA and DNA from a sample exist on the market.

In order to increase the amount of target nucleic acid for hybridization and thereby the hybridization signal obtained (i.e. to improve the sensitivity of detection), the target nucleic acid is amplified. Also advantageously, amplification allows the incorporation of a label into the amplified nucleic acids, which opens different ways of detecting the hybridization complexes formed, and which may increase the sensitivity of detection again.

In the amplification step, the region(s) of the target nucleic acid that comprise the target sequences to which the oligonucleotide probes used in the method hybridize is/are amplified. Said target sequences may be located in multiple genes of a target genome and/or in multiple regions of a single gene of the target genome. Accordingly, the amplification step may encompass the amplification of one or more genes and/or one or more gene regions of a target genome.

Amplification of said relevant region(s) is achieved using one or more primer pairs that flank said region(s). A "primer" refers to a single stranded oligonucleotide sequence capable of acting as a point of initiation for synthesis of a primer extension product which is complementary to the nucleic acid strand to be copied. The length and the sequence of the primer must be such that they allow to prime the synthesis of the extension products. Preferably the primer is about 5-50 nucleotides long. Specific length and sequence will depend on the complexity of the required DNA or RNA targets, as well as on the conditions of primer use such as temperature and ionic strength. The primer design for primers pairs used in a multiplex amplification reaction has to be optimized so that all primer pairs can work at the same annealing temperature. The design of suitable primers is well known to the skilled person.

Amplification of the one or more relevant regions of the target nucleic acid may be performed in one amplification reaction using one or more (i.e. multiplex amplification reaction) suitable primer pairs. Alternatively, the amplification of multiple relevant regions of the target nucleic acid may be performed in multiple amplification reactions, wherein each amplification reaction may independently use one or more suitable primer pairs. As would be understood by one skilled in the art, the amplification products need to be pooled for hybridization with the pools of oligonucleotide probes in step (c) of the method described herein if multiple amplification reactions are performed.

The amplification method used can be either polymerase chain reaction (PCR; Saiki et al., 1988), ligase chain reaction (LCR; Landgren et al., 1988; Wu & Wallace, 1989; Barany, 1991), nucleic acid sequence-based amplification (NASBA; Guatelli et al., 1990; Compton, 1991), transcription-based amplification system (TAS; Kwoh et al., 1989), strand displacement amplification (SDA; Duck, 1990; Walker et al., 1 992) or amplification by means of Q13 replicase (Lizardi et al., 1988; Lomeli et al., 1989) or any other suitable method to amplify nucleic acid molecules known in the art. In preferred embodiments, the target nucleic acid is amplified by a polymerase chain reaction (PCR).

Labelling of the amplification products may be carried out by the use of labelled nucleotides incorporated during the polymerase step of the amplification such as illustrated by Saiki et al. (1 988) or Bej et al. (1990) or by the use of labelled primers, or by any other method known to the person skilled in the art. The nature of the label may be isotopic (³²P, ³⁵S, etc.) or non-isotopic (biotin, digoxigenin, etc.).

The oligonucleotides used as primers or probes may also comprise nucleotide analogues such as phosphorothiates, alkylphosphorothiates or peptide nucleic acids or may contain intercalating agents. As most other variations or changes introduced into the wild-type target sequence, these variations will necessitate adaptions with respect to the conditions under which the oligonucleotide should be used to obtain the required specificity and sensitivity. However the eventual results of hybridisation will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

In the method described herein, the oligonucleotide probes are immobilized to a solid support at a known location, and reverse-hybridization is carried out.

The term "solid support" in the current invention refers to any substrate to which an oligonucleotide probe can be coupled, provided that its hybridization characteristics are retained and provided that the background of hybridization remains low. The solid support may be a microtiter plate, a membrane (e.g. nylon or nitrocellulose), a microsphere (bead) or a microarray (e.g. glass, silicon, plastic). Prior to application to the membrane or fixation it may be convenient to modify the oligonucleotide probe in order to facilitate fixation or to improve the hybridization efficiency. Such modifications may encompass homopolymer tailing, coupling with different reactive groups such as aliphatic groups, NH₂ groups, SH groups, or carboxylic groups, or coupling with biotin, haptens or proteins. For example, the oligonucleotide probes used in the herein described methods may be immobilized to a solid support by means of a homopolymer tailing sequence (e.g. polyT) which is added at the 3' or 5' extremity of the probe. Said tailing may be done during chemical synthesis of the oligonucleotide, usually resulting in a 5' polyT tail, or afterwards, e.g. via enzymatic tailing using terminal deoxynucleotidyl transferase (Pharmacia), resulting in a 3' polyT tail.

The oligonucleotide probes are immobilized to a solid support in known locations (dots, lines or other figures). In a preferred embodiment, oligonucleotide probes are immobilized to a membrane strip in a line fashion such as in a line probe assay (LiPA assay; Stuyver et al. 1993; WO 94/12670).

The probes may be immobilized individually or as mixtures to delineated locations on the solid support. In the method described herein, the oligonucleotide probes of the pools of oligonucleotide probes are immobilized as mixtures to delineated locations on a first solid support for the first hybridization, whereas in the second hybridization, the oligonucleotide probes are immobilized individually to delineated locations on one or more second solid supports.

Detection of the hybridization complexes or hybrids formed may be done according to any methods known in the art, the type of detection of course depending on the type of label used. When biotin is used as a label, streptavidin conjugated detection agents may be used, such as e.g. streptavidin conjugated alkaline phosphatase, causing a blue precipitation signal, or streptavidin conjugated horse radish peroxidase, causing a color reaction in solution.

In the method described herein, relevant amplification products of the target nucleic acid are first hybridized with a set or combination of at least two pools of oligonucleotide probes (step (c) of the method). A positive hybridisation signal indicates that the target nucleic acid comprises at least one of the target sequences to which the oligonucleotide probes in the pool hybridize. To identify the particular target sequence, a second hybridization with the individual oligonucleotide probes of the positive pools is carried out. The first hybridization thus determines which second hybridizations with individual oligonucleotide probes need to be carried out to complete the genetic analysis of the target nucleic acid and can thus significantly reduce the number of hybridizations with individual oligonucleotide probes that needs to be performed.

A "pool" of oligonucleotide probes may comprise between 2 and 12 such as 3, 4, 5, 6, 7, 8, 9, 10, 11 or 12 oligonucleotide probes. The oligonucleotide probes in a pool may hybridize to the same or different amplified target nucleic acid regions, preferably different amplified target nucleic acid regions. In certain embodiments, the composition of the pools may be determined by reverse-hybridization assays with individual oligonucleotide probes known in the art, wherein a pool consists of different oligonucleotide probes from one reverse-hybridization assay, such as from one LiPA assay, known in the art.

In embodiments, a pool consists of different oligonucleotide probes from one solid support to which multiple individual oligonucleotide probes are immobilized. In these embodiments, the positive pools from the first hybridization determine which solid supports need to be used in the second hybridization. Different pools may comprise oligonucleotide probes from the same solid support or from different solid supports. In further embodiments, the set or combination of pools of oligonucleotide probes comprises at least 2, preferably at least 3, more preferably at least 4, even more preferably at least 5 or more pools from different solid supports.

The present method is particularly advantageous for the genetic analysis of target nucleic acids that would require more than 2, preferably more than 3, more preferably more than 4, even more preferably more than 5 reverse-hybridization assays with individual oligonucleotide probes to complete the analysis in that the first hybridization determines which reverse-hybridization assays with individual oligonucleotide probes have to be performed in the second hybridization so that reverse-hybridizations assays that would not yield a positive signal are not performed. Accordingly, in preferred embodiments of the method described herein, step (c) is performed using a set of pools of oligonucleotide probes that comprises at least 3, preferably at least 4, more preferably at least 5, even more preferably at least 6 or more pools of oligonucleotide probes that consist of different oligonucleotide probes from one reverse-hybridization assay and that differ in that they comprise oligonucleotide probes from different reverse-hybridization assays.

The herein described method can be applied for analysing genomic DNA for multiple mutations in one or more genes. For many genetic diseases, there is more than one mutation associated with the disease. Non-limiting examples of such multimutational diseases include cystic fibrosis, β-thalassaemia, Tay-Sachs disease, phenylketonuria, Sickle cell anemia, Gaucher's disease and cancers. In addition to the diagnosis of the disease, the method described herein may allow to discriminate between healthy normals and healthy carriers.

In particular embodiments, the method disclosed herein is for the detection of a mutation in the *CFTR* gene and/or the genetic analysis of the *CFTR* gene, said method comprising:
(a) optionally releasing, isolating or concentrating the nucleic acids present in a sample;
(b) amplifying one or more regions of the *CFTR* gene present in the sample with one or more suitable primer pairs, said one or more regions of the *CFTR* gene comprising the target sequences to which the oligonucleotide probes hybridize;
(c) performing a first hybridization reaction with the amplification products obtained in step (b) and a combination of pools of oligonucleotide probes comprising:
   a pool of oligonucleotide probes that permit detection of 2 or more, preferably all, of the *CFTR* mutations 3199del6, 711+1G>T, I507del, S1251N, R560T and R553X;
   a pool of oligonucleotide probes that permit detection of 2 or more, preferably all, of the *CFTR* mutations 3120+1G>A, Q552X, 3905insT, 3272-26A>G and 1898+1G>A;
   a pool of oligonucleotide probes that permit detection of 2 or more, preferably all, of the *CFTR* mutations 2183AA>G, 2184delA, 394delTT, G85E, 2789+5G>A, R1162X, R117H and R347P;
   a pool of oligonucleotide probes that permit detection of 2 or more, preferably all, of the *CFTR* mutations R334W, 1078delT, 3849+10kbC>T, 3659delC, A455E, 2143delT, E60X and 711+5G>A;
   a pool of oligonucleotide probes that permit detection of 2 or more, preferably all, of the *CFTR* mutations 1259insA, 4016insT, 4382delA, E217G, D579G, G1244E, G1349D, I502T, L1065P, R1070Q and R334Q;
   a pool of oligonucleotide probes that permit detection of 2 or more, preferably all, of the *CFTR* mutations R1158W, S549R(A\C), 991del5, D1152H, 1898+3A\G, R1066H, T338I, R347H, 621+3A>G and 852del22;
   a pool of oligonucleotide probes that permit detection of 2 or more, preferably all, of the *CFTR* mutations dele1, dele2(ins182), dele22-24, dele14b-17b, 1706del17, R1066C and S912X;
   a pool of oligonucleotide probes that permit detection of 2 or more, preferably all, of the *CFTR* mutations dele17a-17b-18, E585X, L1077P, dele22,23, dele2 and 1677delTA;
   a pool of oligonucleotide probes that permit detection of 2 or more, preferably all, of the *CFTR* mutations M1V, E92X, 1584+18672A>G, G178R, E822X , L206W, I336K, R352Q and M1101K; and
   a pool of oligonucleotide probes that permit detection of 2 or more, preferably all, of the *CFTR* mutations 1609delCA, D110E, 1811+1,6kbA>G, 2184insA, 712-1G>T, Q890X, Y1092X, L346P and 4374+1G>A, wherein said pools of oligonucleotide probes are immobilized to a known location on a first support;
(d) detecting the hybrids formed in step (c);
(e) performing a second hybridization reaction with the relevant amplification products obtained in step (b) and a combination of the oligonucleotide probes that are present in the pool(s) of oligonucleotide probes for which a positive hybridization signal was detected in step (d), wherein said oligonucleotide probes are individually immobilized to a known location on one or more second support(s);
(f) detecting the hybrids formed in step (e); and
(g) inferring the *CFTR* mutation(s) present in the sample from the differential hybridization signal(s) obtained in step (f).

The *CFTR* mutations referred to herein are according to the Cystic Fibrosis Mutation Database (CFTR1) held by the Cystic Fibrosis Centre at the Hospital for Sick Children in Toronto (http://genet.sickkids.on.ca).

In further particular embodiments, the amplification products obtained in step (b) are hybridized in step (c) with a combination of pools of oligonucleotide probes comprising:
a pool of oligonucleotide probes comprising at least 2, preferably all, oligonucleotide probes selected from the group consisting of: SEQ ID NO:1, SEQ ID NO:2, SEQ ID NO:3, SEQ ID NO:4, SEQ ID NO:5 and SEQ ID NO:6;
a pool of oligonucleotide probes comprising at least 2, preferably all, oligonucleotide probes selected from the group consisting of: SEQ ID NO:7, SEQ ID NO:8, SEQ ID NO:9, SEQ ID NO:10 and SEQ ID NO:11;
a pool of oligonucleotide probes comprising at least 2, preferably all, oligonucleotide probes selected from the group consisting of: SEQ ID NO:12, SEQ ID NO:13, SEQ ID NO:14, SEQ ID NO:15, SEQ ID NO:16, SEQ ID NO:17, SEQ ID NO:18 and SEQ ID NO:19;
a pool of oligonucleotide probes comprising at least 2, preferably all, oligonucleotide probes selected from the group consisting of: SEQ ID NO:20, SEQ ID NO:21, SEQ ID NO:22, SEQ ID NO:23, SEQ ID NO:24, SEQ ID NO:25, SEQ ID NO:26 and SEQ ID NO:27;
a pool of oligonucleotide probes comprising at least 2, preferably all, oligonucleotide probes selected from the group consisting of: SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37 and SEQ ID NO:38;
a pool of oligonucleotide probes comprising at least 2, preferably all, oligonucleotide probes selected from the group consisting of: SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47 and SEQ ID NO:48;
a pool of oligonucleotide probes comprising at least 2, preferably all, oligonucleotide probes selected from the group consisting of: SEQ ID NO:49, SEQ ID NO:50, SEQ ID NO:51, SEQ ID NO:52, SEQ ID NO:53, SEQ ID NO:54 and SEQ ID NO:55;
a pool of oligonucleotide probes comprising at least 2, preferably all, oligonucleotide probes selected from the group consisting of: SEQ ID NO:56, SEQ ID NO:57, SEQ ID NO:58, SEQ ID NO:59, SEQ ID NO:60 and SEQ ID NO:61;
a pool of oligonucleotide probes comprising at least 2, preferably all, oligonucleotide probes selected from the group consisting of: SEQ ID NO:62, SEQ ID NO:63, SEQ ID NO:64, SEQ ID NO:65, SEQ ID NO:66, SEQ ID NO:67, SEQ ID NO:68, SEQ ID NO:69 and SEQ ID NO:70;
a pool of oligonucleotide probes comprising at least 2, preferably all, oligonucleotide probes selected from the group consisting of: SEQ ID NO:71, SEQ ID NO:72, SEQ ID NO:73, SEQ ID NO:74, SEQ ID NO:75, SEQ ID NO:76, SEQ ID NO:77, SEQ ID NO:78 and SEQ ID NO:79.

In yet further particular embodiments, regions of the CFTR gene are amplified in step (b) using the primers set forth in SEQ ID NO:80-165.

The herein described method may also be applied for the simultaneous detection and differentiation of microbial species such as viral, bacterial and/or fungal species present in a particular type of sample such as a blood or serum sample, sputum samples, BAL samples, etc. or an environmental sample, wherein the oligonucleotide probes specifically hybridize to target sequences in viral, bacterial or fungal nucleic acids.

A further application resides in the use of the method described herein for the genotyping of microbial species such as viral, bacterial and/or fungal species present in a sample, wherein the oligonucleotide probes specifically hybridize to target sequences in the viral, bacterial or fungal genome.

Further disclosed herein is a set of arrays for detection of multiple target sequences from a target nucleic acid and/or genetic analysis of a target nucleic acid suitable for use in the method described herein, said set of arrays comprising:
- a first array comprising a first support and at least two pools of oligonucleotide probes immobilized to a known location on said first support; and
- at least two arrays comprising a second support and the oligonucleotide probes that are present in a pool of oligonucleotide probes of the first array individually immobilized to a known location on said second support,
wherein the oligonucleotide probes specifically hybridize to a target sequence from the target nucleic acid, and wherein the oligonucleotide probes are capable of hybridizing to the amplification products under the same hybridization and wash conditions.

Also disclosed herein is a kit for detection of multiple target sequences from a target nucleic acid and/or genetic analysis of a target nucleic acid suitable for use in the method described herein, said kit comprising:
- one or more suitable primer pairs for amplifying the target nucleic acid region(s)comprising the target sequences to which the oligonucleotide probes hybridize;
- a set of arrays as described herein;
- a hybridization buffer, or components necessary for producing said buffer;
- a wash solution, or components necessary for producing said wash solution;
- optionally means for detection of the hybrids.

The term "hybridization buffer" means a buffer enabling a hybridization reaction to occur between the oligonucleotide probes and the amplification products of the target nucleic acid under the appropriate stringency conditions.

The term "wash solution" means a solution enabling washing of the hybrids formed under the appropriate stringency conditions.

As illustrated in the Examples section, an assay was designed for screening for *CFTR* mutations. The principle of the assay is based on reverse-hybridization of amplified nucleic acid fragments such as biotinylated PCR fragments of the *CFTR* gene onto oligonucleotide probes that specifically hybridize with mutant target sequences from the *CFTR* gene. In a first hybridization, the amplification products are first hybridized with a set of pools of these oligonucleotide probes to detect the presence of a mutation in the *CFTR* gene, followed by a second hybridization with the individual oligonucleotide probes of the positive pools to identify the particular mutation. The hybrids can via a biotin-streptavidin coupling be detected with a non-radioactive colour developing system.

The examples of the present invention presented below are provided only for illustrative purposes and not to limit the scope of the invention.

### EXAMPLES

### Example 1: Development of an assay for detecting mutations in the CFTR gene

The INNO-LiPA® *CFTR* tests *CFTR19, CFTR*17+Tn, *CFTR* Italian Regional, *CFTR* Deletions+6 and *CFTR* Extra (Fujirebio Europe, Ghent, Belgium) are line probe assays for the simultaneous *in vitro* detection and identification of human *CFTR* gene mutations and their corresponding wild-type sequences in human biological samples (e.g. whole blood, buccal brush and dried blood spot samples).

A new membrane strip was developed (also referred to herein as *CFTR*iage strip), wherein different oligonucleotide probes from one of the aforementioned INNO-LiPA® *CFTR* tests (i.e. a pool of oligonucleotide probes) were applied - as a mixture - as a line on the membrane strip. The membrane strip comprises different pools of oligonucleotide probes from all aforementioned INNO-LiPA® *CFTR* tests as parallel lines on the membrane. The pools only contain oligonucleotide probes from the INNO-LiPA® *CFTR* tests that specifically hybridize with a mutant target sequence from the *CFTR* gene. The composition of the pools of oligonucleotide probes is specified in Table 1.

**Table 1: Composition of the pools of oligonucleotide probes on the CFTRiage strip.**

| ***CFTR* mutation** | **Probe sequence** | **SEQ ID NO** | **Inno-LiPA® *CFTR* test** | **Pool** |
|---|---|---|---|---|
| 3199del6 | ATAATAAAAGCCACTGGCA | 1 | *CFTR*19 | 1 |
| 711+1G>T | TTTGATGAATTATGTACCT | 2 | *CFTR*19 | 1 |
| I507del | AACACCAAAGATATTTTCTTT | 3 | *CFTR*19 | 1 |
| S1251N | GAACAAAGTATTCTTCCCTG | 4 | *CFTR*19 | 1 |
| R560T | TTTAGCAACGTGAATAACT | 5 | *CFTR*19 | 1 |
| R553X | GTCTTGCTCATTGACCTC | 6 | *CFTR*19 | 1 |
| 3120+1G>A | TTCATCCAGATATGTAAAAAT | 7 | *CFTR*19 | 2 |
| Q552X | GTGGAGGTTAACGAG | 8 | *CFTR*19 | 2 |
| 3905insT | TCAGCTTTTTTTGAGAC | 9 | *CFTR*19 | 2 |
| 3272-26A>G | TGTTATTTGCAGTGTTTT | 10 | *CFTR*19 | 2 |
| 1898+1G>A | TTTGAAAGATATGTTCTTTG | 11 | *CFTR*19 | 2 |
| 2183AA>G | GAAACAAAAAGCAATCTTTT | 12 | *CFTR* 17+Tn | 3 |
| 2184delA | GAAACAAAAAACAATCTTTT | 13 | *CFTR* 17+Tn | 3 |
| 394delTT | GAATCTTTATATTTAGGGGT | 14 | *CFTR* 17+Tn | 3 |
| G85E | GTTATGTTCTATGAAATCTTTTT | 15 | *CFTR* 17+Tn | 3 |
| 2789+5G>A | AAGTGAATATTCCATGTC | 16 | *CFTR* 17+Tn | 3 |
| R1162X | AAAGACTCAGCTCACAG | 17 | *CFTR* 17+Tn | 3 |
| R117H | GGAGGAACACTCTATCG | 18 | *CFTR* 17+Tn | 3 |
| R347P | ATTGTTCTGCCCATGGC | 19 | *CFTR* 17+Tn | 3 |
| R334W | AATATTTTCCAGAGGATGATT | 20 | *CFTR* 17+Tn | 4 |
| 1078delT | AGGGTTCTTGTGGTG | 21 | *CFTR* 17+Tn | 4 |
| 3849+10kbC>T | TAAAATGGTGAGTAAGAC | 22 | *CFTR* 17+Tn | 4 |
| 3659delC | GAAACCTACAAGTCAACC | 23 | *CFTR* 17+Tn | 4 |
| A455E | TTG TTG GAG GTT GCT-T150 | 24 | *CFTR* 17+Tn | 4 |
| 2143delT | GTTTCTCATAGAAGGAG | 25 | *CFTR* 17+Tn | 4 |
| E60X | GATAGATAGCTGGCTT | 26 | *CFTR* 17+Tn | 4 |
| 711+5G>A | GGGGATGAAGTATATACCTATTG | 27 | *CFTR* 17+Tn | 4 |
| 1259insA | GGATTTCTTACAAAAAGC | 28 | *CFTR* Italian regional | 5 |
| 4016insT | AAGTATTTATTTTTTTCTGG | 29 | *CFTR* Italian regional | 5 |
| 4382delA | ACTTTGTTCTCTCTATGAC | 30 | *CFTR* Italian regional | 5 |
| E217G | GGGGGTTGTTACAGGC | 31 | *CFTR* Italian regional | 5 |
| D579G | ATACCTAGGTGTTTTAACA | 32 | *CFTR* Italian regional | 5 |
| G1244E | CTTGGAAAGAACTGGATC | 33 | *CFTR* Italian regional | 5 |
| G1349D | GCCATGACCACAAGC | 34 | *CFTR* Italian regional | 5 |
| I502T | TTTAGTGGTGCCAGG | 35 | *CFTR* Italian regional | 5 |
| L1065P | GCACGAGGTGTCCATA | 36 | *CFTR* Italian regional | 5 |
| R1070Q | GACAGCAGCCTTACTTT | 37 | *CFTR* Italian regional | 5 |
| R334Q | CCTCCAGAAAATATTCACC | 38 | *CFTR* Italian regional | 5 |
| R1158X | TCACAGATCACATCTGAA | 39 | *CFTR* Italian regional | 6 |
| S549R(A>C) | TGGAATCACACTGCGT | 40 | *CFTR* Italian regional | 6 |
| 991del5 | AAATGATTGAAAAGACAGTA | 41 | *CFTR* Italian regional | 6 |
| D1152H | CAGCATACATGTGGATAG | 42 | *CFTR* Italian regional | 6 |
| 1898+3A>G | TATTCAAAGAACACACCT | 43 | *CFTR* Italian regional | 6 |
| R1066H | GGACACTTCATGCCTTC | 44 | *CFTR* Italian regional | 6 |
| T338I | AGATGGTGATGAATATTTT | 45 | *CFTR* Italian regional | 6 |
| R347H | GCATTGTTCTGCACAT | 46 | *CFTR* Italian regional | 6 |
| 621+3A>G | GTGATACTTCCTTGCA | 47 | *CFTR* Italian regional | 6 |
| 852del22 | CTGGGCTAGGTAGCAAC | 48 | *CFTR* Italian regional | 6 |
| dele1 | TAACAAAACATGGTCAGCAA | 49 | *CFTR* Deletions + 6 | 7 |
| dele2(ins182) | GGTTGCAACTTAGACTGGATATG | 50 | *CFTR* Deletions + 6 | 7 |
| dele22-24 | CCTGGTTTATGGATGAGGC | 51 | *CFTR* Deletions + 6 | 7 |
| dele14b-17b | GCAACCTCTACCTTCTGTGTTCC | 52 | *CFTR* Deletions + 6 | 7 |
| 1706del17 | CATGCCAACTATGTGAAAAC | 53 | *CFTR* Deletions + 6 | 7 |
| R1066C | GCACAAAGTGTCCATAGT | 54 | *CFTR* Deletions + 6 | 7 |
| S912X | TAATACTAACTGGTGCTG | 55 | *CFTR* Deletions + 6 | 7 |
| dele17a-17b-18 | CAAAGTTCACTTGGGACAG | 56 | *CFTR* Deletions + 6 | 8 |
| E585X | ACAGAAAAATAAATATTTGAAA | 57 | *CFTR* Deletions + 6 | 8 |
| L1077P | GTTGTGGAACGGAGTTTC | 58 | *CFTR* Deletions + 6 | 8 |
| dele22,23 | CCTGGCCTCAAGTTCTGAAAG | 59 | *CFTR* Deletions + 6 | 8 |
| dele2 | CCAGAACAGTGTCAAAAT | 60 | *CFTR* Deletions + 6 | 8 |
| 1677delTA | TGATGAATAGATACAGAAGCCC | 61 | *CFTR* Deletions + 6 | 8 |
| M1V | CCTCTGCACGGTCTCTC | 62 | *CFTR* Extra | 9 |
| E92X | TTTTGTAGTAAGTCACCA | 63 | *CFTR* Extra | 9 |
| 1584+18672A>G | AGAAATGTACGTACTATTCATT | 64 | *CFTR* Extra | 9 |
| G178R | TAAGTATTAGACAACTTGTT | 65 | *CFTR* Extra | 9 |
| E822X | TTAATTTCTTAACTTATTTC | 66 | *CFTR* Extra | 9 |
| L206W | CACTTGCCAAGGAGC | 67 | *CFTR* Extra | 9 |
| I336K | CGGAAAAAATTCACCAC | 68 | *CFTR* Extra | 9 |
| R352Q | GGTCACTCAGCAATTT | 69 | *CFTR* Extra | 9 |
| M1101K | GTTCCAAAAGAGAATAGAA | 70 | *CFTR* Extra | 9 |
| 1609delCA | CTGTTCTGTTTTCCTGG | 71 | *CFTR* Extra | 10 |
| D110E | TTATCCGGTTCATAGGAA | 72 | *CFTR* Extra | 10 |
| 1811+1,6kbA>G | CCTTACTTACATCTCAAGTA | 73 | *CFTR* Extra | 10 |
| 2184insA | AAAGATTGTTTTTTTTGTTT | 74 | *CFTR* Extra | 10 |
| 712-1G>T | ATTTTCCATGGACTTGC | 75 | *CFTR* Extra | 10 |
| Q890X | CCTTTGTCTTAAAGAGGAGT | 76 | *CFTR* Extra | 10 |
| Y1092X | TTGACAGTTACAAGAACCA | 77 | *CFTR* Extra | 10 |
| L346P | CATTGTTCCGCGCATG | 78 | *CFTR* Extra | 10 |
| 4374+1G>A | CAATTTTTGATGAGTCT | 79 | *CFTR* Extra | 10 |

In addition to the pools of oligonucleotide probes, individual oligonucleotide probes that specifically hybridize with a mutant target sequence from the *CFTR* gene that occurs frequently in the population, in particular at a frequency of more than 1%, and the corresponding wild-type oligonucleotide probes were individually applied to the membrane. Taken altogether, the newly developed strip allows simultaneous detection of 89 mutations, including all mutations with a frequency of more than 1%, representing an overall detection rate of more than 91.8%. This coverage is broader compared to any INNO-LiPA® *CFTR* test. Accordingly, the newly developed strip allows for improved detection of cystic fibrosis amongst other because false negative results will be lower.

Further, individual oligonucleotide probes were immobilized to detect polymorphisms in 5T allele in intron 8 causing congenital bilateral absence of the vas deferens (CBAVD) or male infertility that is present in large amount of male CF patients.

The membrane further comprises a conjugate control line that controls the addition of reactive conjugate and substrate working solution during the detection procedure, and primer solution control lines (PS) that control the amplification of the relevant regions of the *CFTR.*

The marker line allows for the correct orientation of the strip.

Table 2 specifies the locations of the pools and individual oligonucleotide probes, and the control lines on the strip.

**Table 2: Location of the marker line, the conjugate control line, the pools of oligonucleotide probes, the mutant (M) and corresponding wild-type (W) oligonucleotide probes, the polymorphism oligonucleotide probes (5T/7T/9T) and the primer solution (PS) control lines on the strip according to an embodiment of the invention. "PS 1", "PS 2", "PS It", "PS Del" and "PS Ext" detect amplification of the CFTR gene regions by multiplex PCRs using the reagents in INNO-LiPA® AMP CFTR kit (PS 1 and PS2), and the reagents available in the INNO-LiPA® CFTR tests CFTR Italian Regional (PS It), CFTR Deletions+6 (PS Del) and CFTR Extra (PS Ext) (Fujirebio Europe, Ghent, Belgium). The composition of the pools is specified in Table 1.**

| | Marker Line 1 |
|---|---|
| | Conjugate control line |
| 1 | PS 1 control |
| 2 | PS 2 control |
| 3 | PS It control |
| 4 | PS Del control |
| 5 | PS Ext control |
| 6 | Pool 1 |
| 7 | Pool 2 |
| 8 | Pool 3 |
| 9 | Pool 4 |
| 10 | Pool 5 |
| 11 | Pool 6 |
| 12 | Pool 7 |
| 13 | Pool 8 |
| 14 | Pool 9 |
| 15 | Pool 10 |
| 16 | W.F508del |
| 17 | W.G542X |
| 18 | W.G551D |
| 19 | W.N1303K |
| 20 | W1282X |
| 21 | W.621+1G->T |
| 22 | W.1717-1G->A |
| 23 | W.CFTRdele2,3(21kb) |
| 24 | W.L927P |
| 25 | M.F508del |
| 26 | M.G542X |
| 27 | M.G551D |
| 28 | M.N1303K |
| 29 | M.W1282X |
| 30 | M.621+1G->T |
| 31 | M.1717-1G->A |
| 32 | M. dele2,3 (21kb) |
| 33 | M.L927P |
| | |
| 34 | 5T |
| 35 | 7T |
| 36 | 9T |

A methodology for detecting mutations in the *CFTR* gene using the newly developed membrane strip is schematically illustrated in Figure 1. Relevant regions of the *CFTR* gene present in a sample (e.g. whole blood, dried blood spots or buccal brushes) were amplified using the multiplex amplification reagents that are available for/in the aforementioned INNO-LiPA® *CFTR* tests according to the manufacturer's instructions. More particularly, for the INNO-LiPA® CFTR tests CFTR19 and CFTR17+Tn, the INNO-LiPA® AMP CFTR kit is available containing reagents for 2 multiplex amplifications (PCR 1 and PCR 2); the INNO-LiPA® CFTR tests CFTR Italian Regional, CFTR Deletions+6 and CFTR Extra contain multiplex amplification reagents for amplifying the relevant *CFTR* gene regions (PCR It, PCR Del and PCR Ext, resp.). More particularly, the relevant regions of the *CFTR* gene were amplified using the primers of Table 3.

**Table 3: Primers suitable for amplifying regions of the CFTR gene that comprise the target sequences to which the oligonucleotide probes present on the newly developed CFTRiage strip hybridize.**

| **Primer name** | **Primer sequence** | **SEQ ID NO** |
|---|---|---|
| I12i.3-Bio | Bio-CATTCTGCCATACCAACAATGGT | 80 |
| I12i.5-Bio | Bio-GTGAATCGATGTGGTGACCA | 81 |
| I16i.3-Bio | Bio-GCAATAGACAGGACTTCAAC | 82 |
| I16i.5-Bio | Bio-CAGAGAAATTGGTCGTTACT | 83 |
| Ex10i.3-Bio | Bio-CATTCACAGTAGCTTACCCA | 84 |
| EX10i.5-Bio | Bio-GCAGAGTACCTGAAACAGGA | 85 |
| EX11i.3-Bio | Bio-GCACAGATTCTGAGTAACCATAAT | 86 |
| Ex11i.5-Bio | Bio-CAACTGTGGTTAAAGCAATAGTGT | 87 |
| Ex20i.3-Bio | Bio-CTATGAGAAAACTGCACTGGA | 88 |
| Ex20i.5- Bio | Bio-GGTCAGGATTGAAAGTGTGCA | 89 |
| Ex21i.3-Bio | Bio-ATTTCAGTTAGCAGCCTTACC | 90 |
| Ex21i.5-Bio | Bio-AAAATGTTCACAAGGGACTCC | 91 |
| I5i.3-Bio | Bio-CTGTCAAGCCGTGTTCTAGAT | 92 |
| I5.5-Bio | Bio-CGCCTTTCCAGTTGTATAATT | 93 |
| Ex4i.3-Bio | Bio-TTGTACCAGCTCACTACCTA | 94 |
| Ex4i.5- Bio | Bio-GAAGTCACCAAAGCAGTA | 95 |
| Ex2i3-bio | Bio-AGCCACCATACTTGGCTCCT | 96 |
| Ex2i5-Bio | Bio-TCCATATGCCAGAAAAGTTG | 97 |
| Ex3i3-Bio | Bio-CTATTCACCAGATTTCGTAGTCT | 98 |
| Ex3i5-Bio | Bio-CTTGGGTTAATCTCCTTGGATAT | 99 |
| Ex7i3-Bio | Bio-GGTACATTACCTGTATTTTGTTTATTGC | 100 |
| Ex7i5-Bio | Bio-CATGCTCAGATCTTCCATTCCAAGATCC | 101 |
| I8/Ex9i3-Bio | Bio-GGAAGAAAAAGCCACTGAAAATAATATGAG | 102 |
| I8/Ex9i5-Bio | Bio-TAATTGTACATAAAACAAGCATCTATTGAA | 103 |
| Ex13i3-Bio | Bio-CGTATAGAGTTGATTGGATTGAGAATAG | 104 |
| Ex13i5-Bio | Bio-CTTTCGACCAATTTAGTGCAGAAAGAAG | 105 |
| Ex14bi3-Bio | Bio-TGGGAGAAATGAAACAAAGTGG | 106 |
| Ex14bi5-Bio | Bio-GAACACCTAGTACAGCTGCTGG | 107 |
| Ex17Ai3-Bio | Bio-CACCAACTGTGGTAAGATTC | 108 |
| Ex17Ai5-Bio | Bio-ACACACTTTGTCCACTTTGC | 109 |
| I17A/Ex17Bi3-Bio | Bio-TGAGTTCATAGTACCTGTTG | 110 |
| I17A/Ex17bi5-Bio | Bio-CAAAGAATGGCACCAGTGTG | 111 |
| Ex19i3-Bio | Bio-CAGTCTAACAAAGCAAGCAG | 112 |
| Ex19i5-bio | Bio-GCCCGACAAATAACCAAGTG | 113 |
| I19i3-Bio | Bio-GGTATAAGCAGCATATTCTC | 114 |
| I19i5-Bio | Bio-GGCTTCTCAGTGATCTGTTG | 115 |
| I1/I3i3-Bio | Bio-GCATACAAAAAATGGAAGAAAGC | 116 |
| I1/I3i5-Bio | Bio-CTTTAATGGTGTTTACCTACCTAGAG | 117 |
| Ex6ai3-Bio | Bio-GTTTTACTAAAGTGGGCTTTTTGAAAAC | 118 |
| Ex6ai5-Bio | Bio-TAGTTTCTAGGGGTGGAAGATACAATG | 119 |
| Ex6bi3-Bio | Bio-AGGCATTTACCAAACAGTAG | 120 |
| Ex6bi5-Bio | Bio-GCCCATGAAAGTGAATTTGTG | 121 |
| Ex8i3-Bio | Bio-CTTAATCTAGAAAATAAGTGAGTGATCC | 122 |
| Ex8i5-Bio | Bio-AATAGTACAAGGAAGAATCAGTTGTATG | 123 |
| Ex18i3-Bio | Bio-GAAGAGATAAGGATTTAATGACAGATAC | 124 |
| Ex18i5-Bio | Bio-AAGTGTGAATAAAGTCGTTCACAGAAG | 125 |
| Ex22i3-Bio | Bio-AAATGAGCACTGGGTTCATC | 126 |
| Ex22i5-Bio | Bio-ATTCAAATGGTGGCAGGTAG | 127 |
| Ex24i3-Bio | Bio-ACTTAATTCATCCTTGTTTTCTGAGGC | 128 |
| Ex24i5-Bio | Bio-GACATTGCATTCTTTGACTTTTATTTTC | 129 |
| del Ex1.5-Bio | Bio-AGTAGGTCTTTGGCATTAGG | 130 |
| del Ex1.3-Bio | Bio-CCCAGGGTTTCCATATTCTC | 131 |
| Ex1/In1.3-Bio | Bio-CAAGTGCATAGTAGCGTACTTG | 132 |
| del Ex2.5-Bio | Bio-TTAACCTTAGAGCCCTAATTCAG | 133 |
| del Ex2.3-Bio | Bio-AGCCATTTCACATTCCATCATG | 134 |
| del Ex2/Ex3.5-Bio | Bio-CCTTGGTAAAATTAAGCCTCATG | 135 |
| del Ex2ins182.3-Bio | Bio-TGCCTGTTGTTGGTGTAAAC | 136 |
| In3.3-Bio | Bio-GCATACAAAAAATGGAAGAAAGC | 137 |
| In1/Ex2.5-Bio | Bio-TCCATATGCCAGAAAAGTTG | 138 |
| Ex2/In2.3-Bio | Bio-TAGCCACCATACTTGGCTCC | 139 |
| del Ex14b/Ex17b.5-Bio | Bio-CAAATGTTTCCACCTTGAGAAAG | 140 |
| del Ex14b/Ex17b.3-Bio | Bio-CCCTTCAATCACAGAATTG | 141 |
| del Ex17a/Ex17b/Ex18.5-Bio | Bio-TGCCTCATCTGAAATGATTG | 142 |
| del Ex17a/Ex17b/Ex18.3-Bio | Bio-TTTGAGGGCATAGTCCTATC | 143 |
| In17a/Ex17b.5-Bio | Bio-AATATTTCACAGGCAGGAGTC | 144 |
| Ex17b/In17b.3-Bio | Bio-TTGATAACCTATAGAATGCAGC | 145 |
| del Ex22/Ex23.5-Bio | Bio-GGATACCTGGCACGTAATAG | 146 |
| del Ex22/Ex23.3-Bio | Bio-CCTTGTGACTAAACCATAGG | 147 |
| del Ex22/Ex24.5-Bio | Bio-GATTCTGCTGCCACAGATC | 148 |
| del Ex22/Ex24.3-Bio | Bio-CCTGTGCCTATGAAGACAAG | 149 |
| Ex22/In22.3-Bio | Bio-AGGCATAATGATTCTGTTCC | 150 |
| In14b/Ex15.5-Bio | Bio-AACTTTGGCTGCCAAATAAC | 151 |
| Ex15/In15.3-Bio | Bio-AAACCACAGGCCCTATTGATG | 152 |
| In11/Ex12.5-Bio | Bio-TTGTAATGCATGTAGTGAACTGTTTAAG | 153 |
| Ex12/In12.3-Bio | Bio-ATACCAACAATGGTGAACATATTTCTC | 154 |
| In9/Ex10.5-Bio | Bio-AATATACACTTCTGCTTAGGATGATAATTG | 155 |
| Ex10/In10.3-Bio | Bio-TTCACAGTAGCTTACCCATAGAGGAAAC | 156 |
| CFTR pri In3.5v1-Bio | Bio-GTCTTGTGTTGAAATTCTCAGG | 157 |
| CFTR pri In11.5v2-Bio | Bio-GTGTATATAGCCAAGTTATTGTACAG | 158 |
| CFTR pri In11.3-Bio | Bio-AGCAATAGTCTACTGTTGACC | 159 |
| CFTR pri In12.5-Bio | Bio-GTGTACATTTTGATGGGATCC | 160 |
| CFTR pri In12.3-Bio | Bio-AAAGATGAAGACACAGTTCCC | 161 |
| CFTR pri Ex14.5-Bio | Bio-CAGGCAAACTTGACTGAACTGG | 162 |
| CFTR pri In14.3-Bio | Bio-TACATGCTACATATTGCATTCTACTC | 163 |
| CFTR pri In25.5-Bio | Bio-TACTGTTCTGTGATATTATGTGTGG | 164 |
| CFTR pri In26.3-Bio | Bio-AACTATCACATGTGAGATTGTTATC | 165 |

The biotinylated amplification products were pooled and hybridized using the newly developed strip described above. Possible outcomes are illustrated in Figure 1. A positive hybridization signal on a line with pooled oligonucleotide probes indicates which INNO-LiPA® *CFTR* tests to perform in the second hybridization. The relevant amplification products were re-used in said second hybridization. Since only two mutations have to be detected to diagnose cystic fibrosis, a maximum of 3 strips had to be used (i.e. the newly developed strip with pools of oligonucleotide probes and maximal 2 strips with individual oligonucleotide probes). The newly developed assay is thus more efficient for the genetic analysis of the *CFTR* gene in that less INNO-LiPA® *CFTR* tests have to be performed to complete the genetic analysis. For the most frequent mutations, individual oligonucleotide probes were immobilized on the new strip such that only 1 strip, the newly developed strip, was needed to diagnose cystic fibrosis in a patient with 2 frequent mutations.
More specific test cases are illustrated in examples 2-4.

### Example 2: Test case L10016

Sample L10016 was processed as described in Example 1.

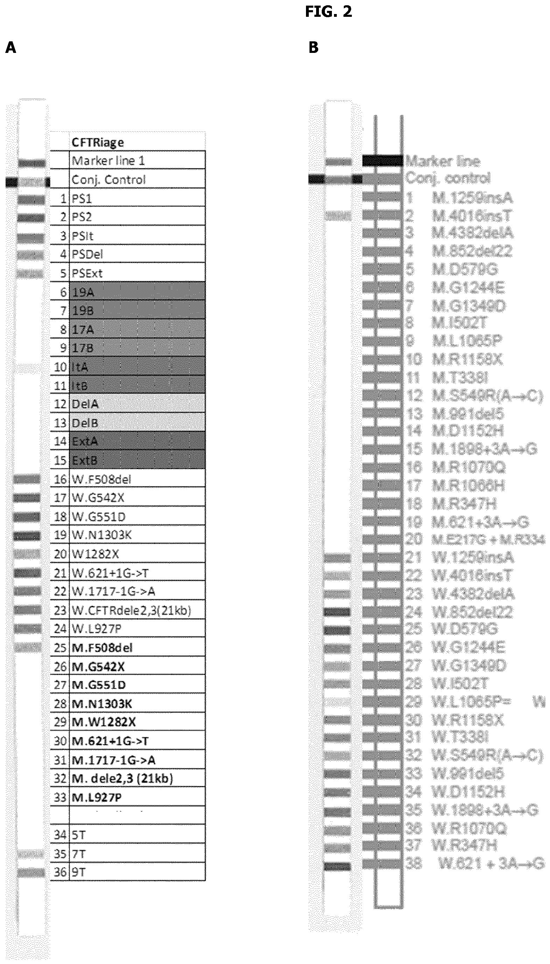

Briefly, the *CFTR* gene present in the sample was amplified using the primers of Table 3 by multiplex PCRs. The biotinylated amplification products were pooled and hybridized using the newly developed strip (*CFTR*iage strip) described in example 1 above. The outcome of the first hybridization is shown in Figure 2A.

The conjugate control was positive, indicating that the assay run was ok.

The 5 amplification control lines (PS 1, PS 2, PS It, PS Del and PS Ext) were positive indicating that the corresponding multiplex PCRs were ok and that the amplification products were added to the stip.

Line 10 corresponding to a pool of oligonucleotide probes from the INNO-LiPA® CFTR Italian Regional test was positive, indicating that the sample was positive for a *CFTR* mutation, but a second hybridization with the INNO-LiPA® CFTR Italian Regional test was required to identify the mutation.

Line 25 was positive, indicating that the sample was positive for *CFTR* mutation F508del.

Line 16 corresponding to the wild-type line for F508del was positive, indicating that F508del was not homozygous.

Lines 35 and 36 were positive, indicating that the sample was positive for the 7T and 9T polymorphisms.

The results of the first strip indicated that sample L10016 was positive for F508del and an Italian mutation and that a second hybridization with the INNO-LiPA® CFTR Italian Regional strip was required to confirm and identify the Italian mutation.

At least the biotinylated amplification products obtained with the multiplex PCR reagents of the INNO-LiPA® CFTR Italian Regional test were hybridized with the INNO-LiPA® CFTR Italian Regional strip. The results of said second hybridization are shown in Figure 2B.

The conjugate control was positive, indicating that the assay run was ok.

Lines 2 and 22 were positive indicating that the sample L10016 was heterozygous for 4016insT.

In conclusion, after 2 hybridizations, it was concluded that sample L10016 contained the *CFTR* mutations F508del and 4016insT.

### Example 3: Test case L12711

Sample L12711 was processed as described in Example 1.

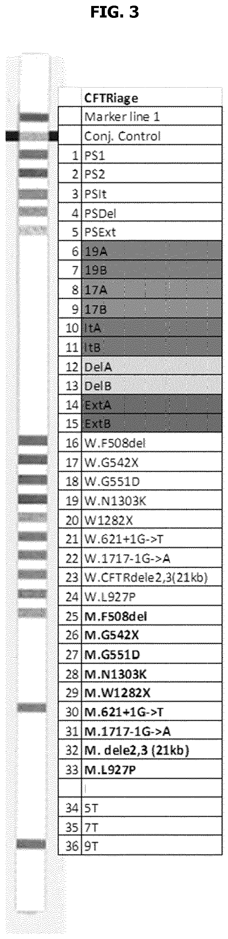

Briefly, the *CFTR* gene present in the sample was amplified using the primers of Table 3 by multiplex PCRs. The biotinylated amplification products were pooled and hybridized using the newly developed strip (*CFTR*iage strip) described in example 1 above. The outcome of the first hybridization is shown in Figure 3.

The conjugate control was positive, indicating that the assay run was ok.

The 5 amplification control lines (PS 1, PS 2, PS It, PS Del and PS Ext) were positive indicating that the corresponding multiplex PCRs were ok and that the amplification products were added to the stip.

Line 25 was positive, indicating that the sample was positive for mutation F508del.

Line 30 was positive, indicating that the sample was positive for mutation 621+1G->T.

Line 16 corresponding to the wild-type line for F508del was positive, indicating that F508del was not homozygous.

Line 21 corresponding to the wild-type line for 621+1G->T was positive, indicating that 621+1G->T was not homozygous.

Line 36 was positive, indicating that the sample was positive for the 9T polymorphism.

The results of the newly developed strip indicated that sample L12711 was positive for F508del and 621+1G->T. The *CFTR* gene analysis was completed using the newly developed strip; no further hybridizations were required.

### Example 4 Test case NA11496

Sample NA11496 was processed as described in Example 1.

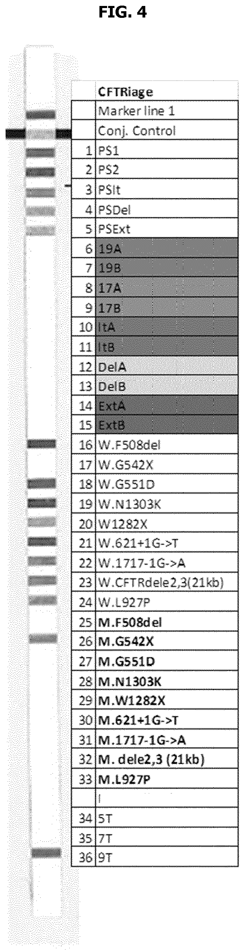

Briefly, the *CFTR* gene present in the sample was amplified using the primers of Table 3 by multiplex PCRs. The biotinylated amplification products were pooled and hybridized using the newly developed strip (*CFTR*iage strip) described in example 1 above. The outcome of the first hybridization is shown in Figure 4.

The conjugate control was positive, indicating that the assay run was ok.

The 5 amplification control lines (PS 1, PS 2, PS It, PS Del and PS Ext) were positive indicating that the corresponding multiplex PCRs were ok and that the amplification products were added to the stip.

Line 26 was positive, indicating that the sample was positive for mutation G542X.

Line 17 corresponding to the wild-type line for G542X was negative, indicating that G542X was homozygous.

Line 36 was positive, indicating that the sample was positive for the 9T polymorphism.

The results of the newly developed strip indicated that sample NA11496 was homozygous for mutation G542X. The *CFTR* gene analysis was completed using the newly developed strip; no further hybridizations were required.

## Claims

1. A method for detection of multiple target sequences from a target nucleic acid and/or genetic analysis of a target nucleic acid in a sample, comprising:
(a) optionally releasing, isolating or concentrating the nucleic acids present in the sample;
(b) amplifying one or more regions of the target nucleic acid present in the sample with one or more suitable primer pairs, said one or more regions of the target nucleic acid comprising the target sequences to which the oligonucleotide probes hybridize;
(c) performing a first hybridization reaction with the amplification products obtained in step (b) and a combination of at least two pools of oligonucleotide probes, wherein the oligonucleotide probes specifically hybridize to a target sequence from the target nucleic acid, wherein the oligonucleotide probes are capable of hybridizing to the amplification products under the same hybridization and wash conditions, and wherein said pools of oligonucleotide probes are immobilized to a known location on a first support;
(d) detecting the hybrids formed in step (c);
(e) performing a second hybridization reaction with the relevant amplification products obtained in step (b) and a combination of the oligonucleotide probes that are present in the pool(s) of oligonucleotide probes for which a positive hybridization signal was detected in step (d), wherein said oligonucleotide probes are individually immobilized to a known location on one or more second support(s);
(f) detecting the hybrids formed in step (e); and
(g) inferring the presence of at least one of the multiple target sequences from the target nucleic acid in the sample and/or the genotype of the target nucleic acid present in the sample from the differential hybridization signal(s) obtained in step (f).

2. The method according to claim 1, wherein in step (c) the amplification products are hybridized with a combination of at least three, four, five or more pools of oligonucleotide probes.

3. The method according to claim 1 or 2, wherein the support is a solid support, preferably a membrane strip.

4. The method according to claim 3, wherein the pools of oligonucleotide probes that are immobilized on the first support and the oligonucleotides probes that are individually immobilized on the one or more second support(s) are immobilized on a membrane strip in a line fashion.

5. The method according to any one of claims 1 to 4, wherein the target sequence is a mutant target sequence, a polymorphic target sequence or a wild-type target sequence.

6. The method according to any one of claims 1 to 5, wherein the first support further comprises individual oligonucleotide probes that specifically hybridize to a target sequence from the target nucleic acid, wherein said individual oligonucleotide probes are individually applied to a known location on said first support.

7. The method according to any one of claims 1 to 6, wherein said first and second supports further comprise positive control oligonucleotide probes immobilized to a known location on said supports, wherein said positive control oligonucleotide probes hybridize to the amplification products obtained in step (b).

8. The method according to any one of claims 1 to 7, wherein the target nucleic acid is genomic DNA.

9. The method according to claim 8, wherein the target sequences are located within multiple genes present in said genomic DNA.

10. The method according to claim 8, wherein the target sequences are located within multiple regions of a gene present in said genomic DNA.

11. The method according to claim 10, wherein the gene is the cystic fibrosis transmembrane regulator (*CFTR*) gene.

12. The method according to claim 11, wherein the oligonucleotide probes in the pools of oligonucleotide probes specifically hybridize to mutant target sequences from the *CFTR* gene.

13. The method according to claim 12, wherein in step (c) the amplification products are hybridized to a combination of pools of oligonucleotide probes comprising:
a pool of oligonucleotide probes that permit detection of 2 or more, preferably all, of the *CFTR* mutations 3199del6, 711+1G>T, 1507del, S1251N, R560T and R553X;
a pool of oligonucleotide probes that permit detection of 2 or more, preferably all, of the *CFTR* mutations 3120+1G>A, Q552X, 3905insT, 3272-26A>G and 1898+1G>A;
a pool of oligonucleotide probes that permit detection of 2 or more, preferably all, of the *CFTR* mutations 2183AA>G, 2184delA, 394delTT, G85E, 2789+5G>A, R1162X, R117H and R347P;
a pool of oligonucleotide probes that permit detection of 2 or more, preferably all, of the *CFTR* mutations R334W, 1078delT, 3849+10kbC>T, 3659delC, A455E, 2143delT, E60X and 711+5G>A;
a pool of oligonucleotide probes that permit detection of 2 or more, preferably all, of the *CFTR* mutations 1259insA, 4016insT, 4382delA, E217G, D579G, G1244E, G1349D, I502T, L1065P, R1070Q and R334Q;
a pool of oligonucleotide probes that permit detection of 2 or more, preferably all, of the *CFTR* mutations R1158W, S549R(A\C), 991del5, D1152H, 1898+3A\G, R1066H, T338I, R347H, 621+3A>G and 852del22;
a pool of oligonucleotide probes that permit detection of 2 or more, preferably all, of the *CFTR* mutations dele1, dele2(ins182), dele22-24, dele14b-17b, 1706del17, R1066C and S912X;
a pool of oligonucleotide probes that permit detection of 2 or more, preferably all, of the *CFTR* mutations dele17a-17b-18, E585X, L1077P, dele22,23, dele2 and 1677delTA;
a pool of oligonucleotide probes that permit detection of 2 or more, preferably all, of the *CFTR* mutations M1V, E92X, 1584+18672A>G, G178R, E822X , L206W, I336K, R352Q and M1101K; and
a pool of oligonucleotide probes that permit detection of 2 or more, preferably all, of the *CFTR* mutations 1609delCA, D110E, 1811+1,6kbA>G, 2184insA, 712-1G>T, Q890X, Y1092X, L346P and 4374+1G>A.

14. A set of arrays for detection of multiple target sequences from a target nucleic acid and/or genetic analysis of a target nucleic acid according to any one of claims 1 to 13, comprising:
- a first array comprising a first support and at least two pools of oligonucleotide probes immobilized to a known location on said first support; and
- at least two arrays comprising a second support and the oligonucleotide probes that are present in a pool of oligonucleotide probes of the first array individually immobilized to a known location on said second support,
wherein the oligonucleotide probes specifically hybridize to a target sequence from the target nucleic acid, and wherein the oligonucleotide probes are capable of hybridizing to the amplification products under the same hybridization and wash conditions.

15. A kit for detection of multiple target sequences from a target nucleic acid and/or genetic analysis of a target nucleic acid according to any one of claims 1 to 13, comprising:
- one or more suitable primer pairs for amplifying the target nucleic acid region()s comprising the target sequences to which the oligonucleotide probes hybridize;
- a set of arrays according to claim 14;
- a hybridization buffer, or components necessary for producing said buffer;
- a wash solution, or components necessary for producing said wash solution;
- optionally means for detection of the hybrids.

16. A support for detection of multiple mutant target sequences from a CFTR gene, said support comprising pools of oligonucleotide probes immobilized to a known location on said support, wherein said pools of oligonucleotide probes comprise:
a pool of oligonucleotide probes that permit detection of 2 or more, preferably all, of the CFTR mutations 3199del6, 711+1G>T, I507del, S1251N, R560T and R553X;
a pool of oligonucleotide probes that permit detection of 2 or more, preferably all, of the *CFTR* mutations 3120+1G>A, Q552X, 3905insT, 3272-26A>G and 1898+1G>A;
a pool of oligonucleotide probes that permit detection of 2 or more, preferably all, of the *CFTR* mutations 2183AA>G, 2184delA, 394delTT, G85E, 2789+5G>A, R1162X, R117H and R347P;
a pool of oligonucleotide probes that permit detection of 2 or more, preferably all, of the *CFTR* mutations R334W, 1078delT, 3849+10kbC>T, 3659delC, A455E, 2143delT, E60X and 711+5G>A;
a pool of oligonucleotide probes that permit detection of 2 or more, preferably all, of the *CFTR* mutations 1259insA, 4016insT, 4382delA, E217G, D579G, G1244E, G1349D, I502T, L1065P, R1070Q and R334Q;
a pool of oligonucleotide probes that permit detection of 2 or more, preferably all, of the *CFTR* mutations R1158W, S549R(A\C), 991del5, D1152H, 1898+3A\G, R1066H, T338I, R347H, 621+3A>G and 852del22;
a pool of oligonucleotide probes that permit detection of 2 or more, preferably all, of the *CFTR* mutations dele1, dele2(ins182), dele22-24, dele14b-17b, 1706del17, R1066C and S912X;
a pool of oligonucleotide probes that permit detection of 2 or more, preferably all, of the *CFTR* mutations dele17a-17b-18, E585X, L1077P, dele22,23, dele2 and 1677delTA;
a pool of oligonucleotide probes that permit detection of 2 or more, preferably all, of the *CFTR* mutations M1V, E92X, 1584+18672A>G, G178R, E822X, L206W, I336K, R352Q and M1101K; and
a pool of oligonucleotide probes that permit detection of 2 or more, preferably all, of the *CFTR* mutations 1609delCA, D110E, 1811+1,6kbA>G, 2184insA, 712-1G>T, Q890X, Y1092X, L346P and 4374+1G>A.

## Patentansprüche

1. Verfahren zum Nachweisen mehrerer Zielsequenzen aus einer Zielnukleinsäure und/oder zum genetischen Analysieren einer Zielnukleinsäure in einer Probe, umfassend:
(a) optional Freisetzen, Isolieren oder Konzentrieren der in der Probe vorhandenen Nukleinsäuren;
(b) Amplifizieren einer oder mehrerer Regionen der in der Probe vorhandenen Zielnukleinsäure mit einem oder mehreren geeigneten Primerpaaren, wobei die eine oder die mehreren Regionen der Zielnukleinsäure die Zielsequenzen umfassen, mit denen die Oligonukleotidsonden hybridisieren;
(c) Durchführen einer ersten Hybridisierungsreaktion mit den in Schritt (b) erhaltenen Amplifikationsprodukten und einer Kombination von mindestens zwei Pools von Oligonukleotidsonden, wobei die Oligonukleotidsonden spezifisch mit einer Zielsequenz aus der Zielnukleinsäure hybridisieren, wobei die Oligonukleotidsonden in der Lage sind, unter den gleichen Hybridisierungs- und Waschbedingungen mit den Amplifikationsprodukten zu hybridisieren, und wobei die Pools von Oligonukleotidsonden an eine bekannte Stelle auf einem ersten Träger immobilisiert sind;
(d) Nachweisen der in Schritt (c) ausgebildeten Hybride;
(e) Durchführen einer zweiten Hybridisierungsreaktion mit den in Schritt (b) erhaltenen relevanten Amplifikationsprodukten und einer Kombination der Oligonukleotidsonden, die in dem/den Pool(s) von Oligonukleotidsonden vorhanden sind, für die in Schritt (d) ein positives Hybridisierungssignal nachgewiesen wurde, wobei die Oligonukleotidsonden einzeln an einer bekannten Stelle auf einem oder mehreren zweiten Trägern immobilisiert sind;
(f) Nachweisen der in Schritt (e) ausgebildeten Hybride; und
(g) Ableiten des Vorhandenseins mindestens einer der mehreren Zielsequenzen aus der Zielnukleinsäure in der Probe und/oder des Genotyps der in der Probe vorhandenen Zielnukleinsäure aus dem/den in Schritt (f) erhaltenen differentiellen Hybridisierungssignal(en).

2. Verfahren nach Anspruch 1, wobei in Schritt (c) die Amplifikationsprodukte mit einer Kombination aus mindestens drei, vier, fünf oder mehr Pools von Oligonukleotidsonden hybridisiert werden.

3. Verfahren nach Anspruch 1 oder 2, wobei der Träger ein fester Träger ist, vorzugsweise ein Membranstreifen.

4. Verfahren nach Anspruch 3, wobei die Pools von Oligonukleotidsonden, die auf dem ersten Träger immobilisiert sind, und die Oligonukleotidsonden, die einzeln auf dem einen oder den mehreren zweiten Trägern immobilisiert sind, in einer Linie auf einem Membranstreifen immobilisiert sind.

5. Verfahren nach einem der Ansprüche 1 bis 4, wobei die Zielsequenz eine mutierte Zielsequenz, eine polymorphen Zielsequenz oder einer Wildtyp-Zielsequenz ist.

6. Verfahren nach einem der Ansprüche 1 bis 5, wobei der erste Träger ferner einzelne Oligonukleotidsonden umfasst, die spezifisch mit einer Zielsequenz aus der Zielnukleinsäure hybridisieren, wobei die einzelnen Oligonukleotidsonden einzeln an einer bekannten Stelle auf dem ersten Träger aufgebracht werden.

7. Verfahren nach einem der Ansprüche 1 bis 6, wobei der erste und der zweite Träger ferner Positivkontroll-Oligonukleotidsonden umfassen, die an einer bekannten Stelle auf den Trägern immobilisiert sind, wobei die Positivkontroll-Oligonukleotidsonden an die in Schritt (b) erhaltenen Amplifikationsprodukte hybridisieren.

8. Verfahren nach einem der Ansprüche 1 bis 7, wobei die Zielnukleinsäure genomische DNA ist.

9. Verfahren nach Anspruch 8, wobei die Zielsequenzen in mehreren Genen angeordnet sind, die in der genomischen DNA vorliegen.

10. Verfahren nach Anspruch 8, wobei die Zielsequenzen in mehreren Regionen eines Gens angeordnet sind, das in der genomischen DNA vorliegt.

11. Verfahren nach Anspruch 10, wobei das Gen das Mukoviszidose-Transmembranregulator-Gen (*CFTR*) ist.

12. Verfahren nach Anspruch 11, wobei die Oligonukleotidsonden in den Pools von Oligonukleotidsonden spezifisch an mutierte Zielsequenzen aus dem CFTR-Gen hybridisieren.

13. Verfahren nach Anspruch 12, wobei in Schritt (c) die Amplifikationsprodukte mit einer Kombination aus Pools von Oligonukleotidsonden hybridisiert werden, umfassend:
einen Pool von Oligonukleotidsonden, die das Nachweisen von 2 oder mehr, vorzugsweise allen der *CFTR*-Mutationen 3199del6, 711+1G>T, I507del, S1251N, R560T und R553X ermöglichen;
einen Pool von Oligonukleotidsonden, die das Nachweisen von 2 oder mehr, vorzugsweise allen der *CFTR*-Mutationen 3120+1G>A, Q552X, 3905insT, 3272-26A>G und 1898+1G>A ermöglichen;
einen Pool von Oligonukleotidsonden, die das Nachweisen von 2 oder mehr, vorzugsweise allen der *CFTR*-Mutationen 2183AA>G, 2184delA, 394delTT, G85E, 2789+5G>A, R1162X, R117H und R347P ermöglichen;
einen Pool von Oligonukleotidsonden, die das Nachweisen von 2 oder mehr, vorzugsweise allen *CFTR*-Mutationen R334W, 1078delT, 3849+10kbC> T, 3659delC, A455E, 2143delT, E60X und 711+5G> A ermöglichen;
einen Pool von Oligonukleotidsonden, die das Nachweisen von 2 oder mehr, vorzugsweise allen der *CFTR*-Mutationen 1259insA, 4016insT, 4382delA, E217G, D579G, G1244E, G1349D, I502T, L1065P, R1070Q und R334Q ermöglichen;
einen Pool von Oligonukleotidsonden, die das Nachweisen von 2 oder mehr, vorzugsweise allen der *CFTR*-Mutationen R1158W, S549R(A\C), 991del5, D1152H, 1898+3A\G, R1066H, T338I, R347H, 621+3A>G und 852del22 ermöglichen;
einen Pool von Oligonukleotidsonden, die das Nachweisen von 2 oder mehr, vorzugsweise allen der *CFTR*-Mutationen dele1, dele2(ins182), dele22-24, dele14b-17b, 1706del17, R1066C und S912X ermöglichen;
einen Pool von Oligonukleotidsonden, die das Nachweisen von 2 oder mehr, vorzugsweise allen der *CFTR*-Mutationen dele17a-17b-18, E585X, L1077P, dele22,23, dele2 und 1677delTA ermöglichen;
einen Pool von Oligonukleotidsonden, die das Nachweisen von 2 oder mehr, vorzugsweise allen der *CFTR*-Mutationen M1V, E92X, 1584+18672A>G, G178R, E822X, L206W, I336K, R352Q und M1101K ermöglichen; und
einen Pool von Oligonukleotidsonden, die das Nachweisen von 2 oder mehr, vorzugsweise allen der *CFTR*-Mutationen 1609delCA, D110E, 1811+1,6kbA>G, 2184insA, 712-1G>T, Q890X, Y1092X, L346P und 4374+1G>A ermöglichen.

14. Satz von Arrays zum Nachweisen mehrerer Zielsequenzen aus einer Zielnukleinsäure und/oder zum genetischen Analysieren einer Zielnukleinsäure nach einem der Ansprüche 1 bis 13, umfassend:
- ein erstes Array, das einen ersten Träger und mindestens zwei Pools von Oligonukleotidsonden umfasst, die an einer bekannten Stelle auf dem ersten Träger immobilisiert sind; und
- mindestens zwei Arrays, umfassend einen zweiten Träger und die Oligonukleotidsonden, die in einem Pool von Oligonukleotidsonden des ersten Arrays vorhanden sind, die einzeln an einer bekannten Stelle auf dem zweiten Träger immobilisiert sind;
wobei die Oligonukleotidsonden spezifisch mit einer Zielsequenz aus der Zielnukleinsäure hybridisieren, und wobei die Oligonukleotidsonden in der Lage sind, unter den gleichen Hybridisierungs- und Waschbedingungen mit den Amplifikationsprodukten zu hybridisieren.

15. Kit zum Nachweisen mehrerer Zielsequenzen aus einer Zielnukleinsäure und/oder zum genetischen Analysieren einer Zielnukleinsäure nach einem der Ansprüche 1 bis 13, umfassend:
- ein oder mehrere geeignete Primerpaare zum Amplifizieren der Zielnukleinsäureregion(en), die die Zielsequenzen umfassen, mit denen die Oligonukleotidsonden hybridisieren;
- einen Satz von Arrays nach Anspruch 14;
- einen Hybridisierungspuffer oder Komponenten, die zum Herstellen des Puffers erforderlich sind;
- eine Waschlösung oder Komponenten, die zum Herstellen des Waschlösungs erforderlich sind;
- optional Mittel zum Nachweisen der Hybriden.

16. Träger zum Nachweisen mehrerer mutierter Zielsequenzen aus einem CFTR-Gen, wobei der Träger Pools von Oligonukleotidsonden umfasst, die an einer bekannten Stelle auf dem Träger immobilisiert sind, wobei die Pools von Oligonukleotidsonden umfassen:
einen Pool von Oligonukleotidsonden, die das Nachweisen von 2 oder mehr, vorzugsweise allen der *CFTR*-Mutationen 3199del6, 711+1G>T, I507del, S1251N, R560T und R553X ermöglichen;
einen Pool von Oligonukleotidsonden, die das Nachweisen von 2 oder mehr, vorzugsweise allen der *CFTR*-Mutationen 3120+1G>A, Q552X, 3905insT, 3272-26A>G und 1898+1G>A ermöglichen;
einen Pool von Oligonukleotidsonden, die das Nachweisen von 2 oder mehr, vorzugsweise allen der *CFTR*-Mutationen 2183AA>G, 2184delA, 394delTT, G85E, 2789+5G>A, R1162X, R117H und R347P ermöglichen;
einen Pool von Oligonukleotidsonden, die das Nachweisen von 2 oder mehr, vorzugsweise allen *CFTR*-Mutationen R334W, 1078delT, 3849+10kbC> T, 3659delC, A455E, 2143deIT, E60X und 711+5G> A ermöglichen;
einen Pool von Oligonukleotidsonden, die das Nachweisen von 2 oder mehr, vorzugsweise allen der *CFTR*-Mutationen 1259insA, 4016insT, 4382delA, E217G, D579G, G1244E, G1349D, I502T, L1065P, R1070Q und R334Q ermöglichen;
einen Pool von Oligonukleotidsonden, die das Nachweisen von 2 oder mehr, vorzugsweise allen der *CFTR*-Mutationen R1158W, S549R(A\C), 991del5, D1152H, 1898+3A\G, R1066H, T338I, R347H, 621+3A>G und 852del22 ermöglichen;
einen Pool von Oligonukleotidsonden, die das Nachweisen von 2 oder mehr, vorzugsweise allen der *CFTR*-Mutationen dele1, dele2(ins182), dele22-24, dele14b-17b, 1706del17, R1066C und S912X ermöglichen;
einen Pool von Oligonukleotidsonden, die das Nachweisen von 2 oder mehr, vorzugsweise allen der *CFTR*-Mutationen dele17a-17b-18, E585X, L1077P, dele22,23, dele2 und 1677delTA ermöglichen;
einen Pool von Oligonukleotidsonden, die das Nachweisen von 2 oder mehr, vorzugsweise allen der *CFTR*-Mutationen M1V, E92X, 1584+18672A>G, G178R, E822X, L206W, I336K, R352Q und M1101K ermöglichen; und
einen Pool von Oligonukleotidsonden, die das Nachweisen von 2 oder mehr, vorzugsweise allen der *CFTR*-Mutationen 1609delCA, D110E, 1811+1,6kbA>G, 2184insA, 712-1G>T, Q890X, Y1092X, L346P und 4374+1G>A ermöglichen.

## Revendications

1. Procédé de détection de séquences cibles multiples d'un acide nucléique cible et/ou d'analyse génétique d'un acide nucléique cible dans un échantillon, comprenant :
(a) facultativement la libération, l'isolement ou la concentration des acides nucléiques présents dans l'échantillon ;
(b) l'amplification d'une ou plusieurs régions de l'acide nucléique cible présent dans l'échantillon avec une ou plusieurs paires d'amorces appropriées, lesdites une ou plusieurs régions de l'acide nucléique cible comprenant les séquences cibles auxquelles les sondes oligonucléotidiques s'hybrident ;
(c) la conduite d'une première réaction d'hybridation avec les produits d'amplification obtenus dans l'étape (b) et une combinaison d'au moins deux groupes de sondes oligonucléotidiques, dans lequel les sondes oligonucléotidiques s'hybrident spécifiquement à une séquence cible de l'acide nucléique cible, dans lequel les sondes oligonucléotidiques sont capables de s'hybrider aux produits d'amplification dans les mêmes conditions d'hybridation et de lavage, et dans lequel lesdits groupes de sondes oligonucléotidiques sont immobilisés à un emplacement connu sur un premier support ;
(d) la détection des hybrides formés dans l'étape (c) ;
(e) la conduite d'une deuxième réaction d'hybridation avec les produits d'amplification appropriés obtenus dans l'étape (b) et une combinaison des sondes oligonucléotidiques qui sont présentes dans le(s) groupe(s) de sondes oligonucléotidiques pour lesquels un signal d'hybridation positif a été détecté dans l'étape (d), dans lequel lesdites sondes oligonucléotidiques sont immobilisées individuellement à un emplacement connu sur un ou plusieurs deuxièmes supports ;
(f) la détection des hybrides formés dans l'étape (e) ; et
(g) la déduction de la présence d'au moins une des séquences cibles multiples dans l'acide nucléique cible dans l'échantillon et/ou du génotype de l'acide nucléique cible présent dans l'échantillon à partir du ou des signaux d'hybridation différentiels obtenus dans l'étape (f).

2. Procédé selon la revendication 1, dans lequel, dans l'étape (c), les produits d'amplification sont hybridés avec une combinaison d'au moins trois, quatre, cinq ou plus de cinq groupes de sondes oligonucléotidiques.

3. Procédé selon la revendication 1 ou 2, dans lequel le support est un support solide, de préférence une bande de membrane.

4. Procédé selon la revendication 3, dans lequel les groupes de sondes oligonucléotidiques qui sont immobilisés sur le premier support et les sondes oligonucléotidiques qui sont immobilisées individuellement sur les un ou plusieurs deuxièmes supports sont immobilisées sur une bande de membrane de façon linéaire.

5. Procédé selon l'une quelconque des revendications 1 à 4, dans lequel la séquence cible est une séquence cible mutante, une séquence cible polymorphe ou une séquence cible de type sauvage.

6. Procédé selon l'une quelconque des revendications 1 à 5, dans lequel le premier support comprend en outre des sondes oligonucléotidiques individuelles qui s'hybrident spécifiquement à une séquence cible de l'acide nucléique cible, dans lequel lesdites sondes oligonucléotidiques individuelles sont appliquées individuellement à un emplacement connu sur ledit premier support.

7. Procédé selon l'une quelconque des revendications 1 à 6, dans lequel lesdits premier et deuxième supports comprennent en outre des sondes oligonucléotidiques témoins positifs immobilisées à un emplacement connu sur lesdits supports, dans lequel lesdites sondes oligonucléotidiques témoins positifs s'hybrident aux produits d'amplification obtenus dans l'étape (b).

8. Procédé selon l'une quelconque des revendications 1 à 7, dans lequel l'acide nucléique cible est un ADN génomique.

9. Procédé selon la revendication 8, dans lequel les séquences cibles sont situées dans des gènes multiples présents dans ledit ADN génomique.

10. Procédé selon la revendication 8, dans lequel les séquences cibles sont situées dans des régions multiples d'un gène présent dans ledit ADN génomique.

11. Procédé selon la revendication 10, dans lequel le gène est le gène de régulateur transmembranaire de la mucoviscidose (*CFTR*).

12. Procédé selon la revendication 11, dans lequel les sondes oligonucléotidiques dans les groupes de sondes oligonucléotidiques s'hybrident spécifiquement à des séquences cibles mutantes du gène *CFTR.*

13. Procédé selon la revendication 12, dans lequel, dans l'étape (c), les produits d'amplification sont hybridés à une combinaison de groupes de sondes oligonucléotidiques comprenant :
un groupe de sondes oligonucléotidiques qui permettent la détection de 2 ou plus, de préférence la totalité, des mutations de *CFTR* 3199del6, 711+1G>T, I507del, S1251N, R560T et R553X ;
un groupe de sondes oligonucléotidiques qui permettent la détection de 2 ou plus, de préférence la totalité, des mutations de *CFTR* 3120+1G>A, Q552X, 3905insT, 3272-26A>G et 1898+1G>A ;
un groupe de sondes oligonucléotidiques qui permettent la détection de 2 ou plus, de préférence la totalité, des mutations de *CFTR* 2183AA>G, 2184delA, 394delTT, G85E, 2789+5G>A, R1162X, R117H et R347P ;
un groupe de sondes oligonucléotidiques qui permettent la détection de 2 ou plus, de préférence la totalité, des mutations de *CFTR* R334W, 1078delT, 3849+10kbC>T, 3659delC, A455E, 2143delT, E60X et 711+5G>A ;
un groupe de sondes oligonucléotidiques qui permettent la détection de 2 ou plus, de préférence la totalité, des mutations de *CFTR* 1259insA, 4016insT, 4382delA, E217G, D579G, G1244E, G1349D, I502T, L1065P, R1070Q et R334Q;
un groupe de sondes oligonucléotidiques qui permettent la détection de 2 ou plus, de préférence la totalité, des mutations de *CFTR* R1158W, S549R(A\C), 991del5, D1152H, 1898+3A\G, R1066H, T338I, R347H, 621+3A>G et 852del22 ;
un groupe de sondes oligonucléotidiques qui permettent la détection de 2 ou plus, de préférence la totalité, des mutations de *CFTR* dele1, dele2(ins182), dele22-24, dele14b-17b, 1706del17, R1066C et S912X ;
un groupe de sondes oligonucléotidiques qui permettent la détection de 2 ou plus, de préférence la totalité, des mutations de *CFTR* dele17a-17b-18, E585X, L1077P, dele22,23, dele2 et 1677delTA ;
un groupe de sondes oligonucléotidiques qui permettent la détection de 2 ou plus, de préférence la totalité, des mutations de *CFTR* M1V, E92X, 1584+18672A>G, G178R, E822X, L206W, I336K, R352Q et M1101K; et
un groupe de sondes oligonucléotidiques qui permettent la détection de 2 ou plus, de préférence la totalité, des mutations de *CFTR* 1609delCA, D110E, 1811+1,6kbA>G, 2184insA, 712-1G>T, Q890X, Y1092X, L346P et 4374+1G>A.

14. Ensemble de réseaux pour la détection de séquences cibles multiples d'un acide nucléique cible et/ou l'analyse génétique d'un acide nucléique cible selon l'une quelconque des revendications 1 à 13, comprenant :
- un premier réseau comprenant un premier support et au moins deux groupes de sondes oligonucléotidiques immobilisées à un emplacement connu sur ledit premier support ; et
- au moins deux réseaux comprenant un deuxième support et les sondes oligonucléotidiques qui sont présentes dans un groupe de sondes oligonucléotidiques du premier réseau immobilisées individuellement à un emplacement connu sur ledit deuxième support,
dans lequel les sondes oligonucléotidiques s'hybrident spécifiquement à une séquence cible de l'acide nucléique cible, et dans lequel les sondes oligonucléotidiques sont capables de s'hybrider aux produits d'amplification dans les mêmes conditions d'hybridation et de lavage.

15. Kit de détection de séquences cibles multiples d'un acide nucléique cible et/ou d'analyse génétique d'un acide nucléique cible selon l'une quelconque des revendications 1 à 13, comprenant :
- une ou plusieurs paires d'amorces appropriées pour amplifier la ou les région(s) de l'acide nucléique cible comprenant les séquences cibles auxquelles les sondes oligonucléotidiques s'hybrident ;
- un ensemble de réseaux selon la revendication 14 ;
- un tampon d'hybridation, ou les composants nécessaires pour produire ledit tampon ;
- une solution de lavage, ou les composants nécessaires pour produire ledit solution de lavage;
- facultativement un moyen de détection des hybrides.

16. Support pour la détection de séquences cibles mutantes multiples d'un gène CFTR, ledit support comprenant des groupes de sondes oligonucléotidiques immobilisées à un emplacement connu sur ledit support, dans lequel lesdites groupes de sondes oligonucléotidiques comprennent :
un groupe de sondes oligonucléotidiques qui permettent la détection de 2 ou plus, de préférence la totalité, des mutations de *CFTR* 3199del6, 711+1G>T, I507del, S1251N, R560Tand R553X ;
un groupe de sondes oligonucléotidiques qui permettent la détection de 2 ou plus, de préférence la totalité, des mutations de *CFTR* 3120+1G>A, Q552X, 3905insT, 3272-26A>G et 1898+1G>A ;
un groupe de sondes oligonucléotidiques qui permettent la détection de 2 ou plus, de préférence la totalité, des mutations de *CFTR* 2183AA>G, 2184delA, 394delTT, G85E, 2789+5G>A, R1162X, R117H et R347P ;
un groupe de sondes oligonucléotidiques qui permettent la détection de 2 ou plus, de préférence la totalité, des mutations de *CFTR* R334W, 1078delT, 3849+10kbC>T, 3659delC, A455E, 2143delT, E60X et 711+5G>A ;
un groupe de sondes oligonucléotidiques qui permettent la détection de 2 ou plus, de préférence la totalité, des mutations de *CFTR* 1259insA, 4016insT, 4382delA, E217G, D579G, G1244E, G1349D, I502T, L1065P, R1070Q et R334Q;
un groupe de sondes oligonucléotidiques qui permettent la détection de 2 ou plus, de préférence la totalité, des mutations de *CFTR* R1158W, S549R(A\C), 991del5, D1152H, 1898+3A\G, R1066H, T338I, R347H, 621+3A>G et 852del22 ;
un groupe de sondes oligonucléotidiques qui permettent la détection de 2 ou plus, de préférence la totalité, des mutations de *CFTR* dele1, dele2(ins182), dele22-24, dele14b-17b, 1706del17, R1066C et S912X ;
un groupe de sondes oligonucléotidiques qui permettent la détection de 2 ou plus, de préférence la totalité, des mutations de *CFTR* dele17a-17b-18, E585X, L1077P, dele22,23, dele2 et 1677delTA ;
un groupe de sondes oligonucléotidiques qui permettent la détection de 2 ou plus, de préférence la totalité, des mutations de *CFTR* M1V, E92X, 1584+18672A>G, G178R, E822X, L206W, I336K, R352Q et M1101K; et
un groupe de sondes oligonucléotidiques qui permettent la détection de 2 ou plus, de préférence la totalité, des mutations de *CFTR* 1609delCA, D110E, 1811+1,6kbA>G, 2184insA, 712-1G>T, Q890X, Y1092X, L346P et 4374+1G>A.


### SEQUENCE LISTING

<110> Fujirebio Europe NV
<120> DETECTION AND ANALYSIS OF SEQUENCE VARIATIONS IN A TARGET NUCLEIC ACID
<130> FRBE-009-EP-PRIO
<160> 165
<170> PatentIn version 3.5
<210> 1
   <211> 19
   <212> DNA
   <213> artificial sequence
<220>
   <223> Probe for 3199del6
<400> 1
   ataataaaag ccactggca 19
<210> 2
   <211> 19
   <212> DNA
   <213> artificial sequence
<220>
   <223> Probe for 711+1G>T
<400> 2
   tttgatgaat tatgtacct 19
<210> 3
   <211> 21
   <212> DNA
   <213> artificial sequence
<220>
   <223> Probe for I507del
<400> 3
   aacaccaaag atattttctt t 21
<210> 4
   <211> 20
   <212> DNA
   <213> artificial sequence
<220>
   <223> Probe for S1251N
<400> 4
   gaacaaagta ttcttccctg 20
<210> 5
   <211> 19
   <212> DNA
   <213> artificial sequence
<220>
   <223> Probe for R560T
<400> 5
   tttagcaacg tgaataact 19
<210> 6
   <211> 18
   <212> DNA
   <213> artificial sequence
<220>
   <223> Probe for R553X
<400> 6
   gtcttgctca ttgacctc 18
<210> 7
   <211> 21
   <212> DNA
   <213> artificial sequence
<220>
   <223> Probe for 3120+1G>A
<400> 7
   ttcatccaga tatgtaaaaa t 21
<210> 8
   <211> 15
   <212> DNA
   <213> artificial sequence
<220>
   <223> Probe for Q552X
<400> 8
   gtggaggtta acgag 15
<210> 9
   <211> 17
   <212> DNA
   <213> artificial sequence
<220>
   <223> Probe for 3905insT
<400> 9
   tcagcttttt ttgagac 17
<210> 10
   <211> 18
   <212> DNA
   <213> artificial sequence
<220>
   <223> Probe for 3272-26A>G
<400> 10
   tgttatttgc agtgtttt 18
<210> 11
   <211> 20
   <212> DNA
   <213> artificial sequence
<220>
   <223> Probe for 1898+1G>A
<400> 11
   tttgaaagat atgttctttg 20
<210> 12
   <211> 20
   <212> DNA
   <213> artificial sequence
<220>
   <223> Probe for 2183AA>G
<400> 12
   gaaacaaaaa gcaatctttt 20
<210> 13
   <211> 20
   <212> DNA
   <213> artificial sequence
<220>
   <223> Probe for 2184delA
<400> 13
   gaaacaaaaa acaatctttt 20
<210> 14
   <211> 20
   <212> DNA
   <213> artificial sequence
<220>
   <223> Probe for 394delTT
<400> 14
   gaatctttat atttaggggt 20
<210> 15
   <211> 23
   <212> DNA
   <213> artificial sequence
<220>
   <223> Probe for G85E
<400> 15
   gttatgttct atgaaatctt ttt 23
<210> 16
   <211> 18
   <212> DNA
   <213> artificial sequence
<220>
   <223> Probe for 2789+5G>A
<400> 16
   aagtgaatat tccatgtc 18
<210> 17
   <211> 17
   <212> DNA
   <213> artificial sequence
<220>
   <223> Probe for R1162X
<400> 17
   aaagactcag ctcacag 17
<210> 18
   <211> 17
   <212> DNA
   <213> artificial sequence
<220>
   <223> Probe for R117H
<400> 18
   ggaggaacac tctatcg 17
<210> 19
   <211> 17
   <212> DNA
   <213> artificial sequence
<220>
   <223> Probe for R347P
<400> 19
   attgttctgc ccatggc 17
<210> 20
   <211> 21
   <212> DNA
   <213> artificial sequence
<220>
   <223> Probe for R334W
<400> 20
   aatattttcc agaggatgat t 21
<210> 21
   <211> 15
   <212> DNA
   <213> artificial sequence
<220>
   <223> Probe for 1078delT
<400> 21
   agggttcttg tggtg 15
<210> 22
   <211> 18
   <212> DNA
   <213> artificial sequence
<220>
   <223> Probe for 3849+10kbC>T
<400> 22
   taaaatggtg agtaagac 18
<210> 23
   <211> 18
   <212> DNA
   <213> artificial sequence
<220>
   <223> Probe for 3659delC
<400> 23
   gaaacctaca agtcaacc 18
<210> 24
   <211> 15
   <212> DNA
   <213> artificial sequence
<220>
   <223> Probe for A455E
<400> 24
   ttgttggagg ttgct 15
<210> 25
   <211> 17
   <212> DNA
   <213> artificial sequence
<220>
   <223> Probe for 2143delT
<400> 25
   gtttctcata gaaggag 17
<210> 26
   <211> 16
   <212> DNA
   <213> artificial sequence
<220>
   <223> Probe for E60X
<400> 26
   gatagatagc tggctt 16
<210> 27
   <211> 23
   <212> DNA
   <213> artificial sequence
<220>
   <223> Probe for 711+5G>A
<400> 27
   ggggatgaag tatataccta ttg 23
<210> 28
   <211> 18
   <212> DNA
   <213> artificial sequence
<220>
   <223> Probe for 1259insA
<400> 28
   ggatttctta caaaaagc 18
<210> 29
   <211> 20
   <212> DNA
   <213> artificial sequence
<220>
   <223> Probe for 4016insT
<400> 29
   aagtatttat ttttttctgg 20
<210> 30
   <211> 19
   <212> DNA
   <213> artificial sequence
<220>
   <223> Probe for 4382delA
<400> 30
   actttgttct ctctatgac 19
<210> 31
   <211> 16
   <212> DNA
   <213> artificial sequence
<220>
   <223> Probe for E217G
<400> 31
   gggggttgtt acaggc 16
<210> 32
   <211> 19
   <212> DNA
   <213> artificial sequence
<220>
   <223> Probe for D579G
<400> 32
   atacctaggt gttttaaca 19
<210> 33
   <211> 18
   <212> DNA
   <213> artificial sequence
<220>
   <223> Probe for G1244E
<400> 33
   cttggaaaga actggatc 18
<210> 34
   <211> 15
   <212> DNA
   <213> artificial sequence
<220>
   <223> Probe for G1349D
<400> 34
   gccatgacca caagc 15
<210> 35
   <211> 15
   <212> DNA
   <213> artificial sequence
<220>
   <223> Probe for I502T
<400> 35
   tttagtggtg ccagg 15
<210> 36
   <211> 16
   <212> DNA
   <213> artificial sequence
<220>
   <223> Probe for L1065P
<400> 36
   gcacgaggtg tccata 16
<210> 37
   <211> 17
   <212> DNA
   <213> artificial sequence
<220>
   <223> Probe for R1070Q
<400> 37
   gacagcagcc ttacttt 17
<210> 38
   <211> 19
   <212> DNA
   <213> artificial sequence
<220>
   <223> Probe for R334Q
<400> 38
   cctccagaaa atattcacc 19
<210> 39
   <211> 18
   <212> DNA
   <213> artificial sequence
<220>
   <223> Probe for R1158X
<400> 39
   tcacagatca catctgaa 18
<210> 40
   <211> 16
   <212> DNA
   <213> artificial sequence
<220>
   <223> Probe for S549R(A>C)
<400> 40
   tggaatcaca ctgcgt 16
<210> 41
   <211> 20
   <212> DNA
   <213> artificial sequence
<220>
   <223> Probe for 991del5
<400> 41
   aaatgattga aaagacagta 20
<210> 42
   <211> 18
   <212> DNA
   <213> artificial sequence
<220>
   <223> Probe for D1152H
<400> 42
   cagcatacat gtggatag 18
<210> 43
   <211> 18
   <212> DNA
   <213> artificial sequence
<220>
   <223> Probe for 1898+3A>G
<400> 43
   tattcaaaga acacacct 18
<210> 44
   <211> 17
   <212> DNA
   <213> artificial sequence
<220>
   <223> Probe for R1066H
<400> 44
   ggacacttca tgccttc 17
<210> 45
   <211> 19
   <212> DNA
   <213> artificial sequence
<220>
   <223> Probe for T338I
<400> 45
   agatggtgat gaatatttt 19
<210> 46
   <211> 16
   <212> DNA
   <213> artificial sequence
<220>
   <223> Probe for R347H
<400> 46
   gcattgttct gcacat 16
<210> 47
   <211> 16
   <212> DNA
   <213> artificial sequence
<220>
   <223> Probe for 621+3A>G
<400> 47
   gtgatacttc cttgca 16
<210> 48
   <211> 17
   <212> DNA
   <213> artificial sequence
<220>
   <223> Probe for 852del22
<400> 48
   ctgggctagg tagcaac 17
<210> 49
   <211> 20
   <212> DNA
   <213> artificial sequence
<220>
   <223> Probe for dele1
<400> 49
   taacaaaaca tggtcagcaa 20
<210> 50
   <211> 23
   <212> DNA
   <213> artificial sequence
<220>
   <223> Probe for dele2(ins182)
<400> 50
   ggttgcaact tagactggat atg 23
<210> 51
   <211> 19
   <212> DNA
   <213> artificial sequence
<220>
   <223> Probe for dele22-24
<400> 51
   cctggtttat ggatgaggc 19
<210> 52
   <211> 23
   <212> DNA
   <213> artificial sequence
<220>
   <223> Probe for dele14b-17b
<400> 52
   gcaacctcta ccttctgtgt tcc 23
<210> 53
   <211> 20
   <212> DNA
   <213> artificial sequence
<220>
   <223> Probe for 1706del17
<400> 53
   catgccaact atgtgaaaac 20
<210> 54
   <211> 18
   <212> DNA
   <213> artificial sequence
<220>
   <223> Probe for R1066C
<400> 54
   gcacaaagtg tccatagt 18
<210> 55
   <211> 18
   <212> DNA
   <213> artificial sequence
<220>
   <223> Probe for S912X
<400> 55
   taatactaac tggtgctg 18
<210> 56
   <211> 19
   <212> DNA
   <213> artificial sequence
<220>
   <223> Probe for dele17a-17b-18
<400> 56
   caaagttcac ttgggacag 19
<210> 57
   <211> 22
   <212> DNA
   <213> artificial sequence
<220>
   <223> Probe for E585X
<400> 57
   acagaaaaat aaatatttga aa 22
<210> 58
   <211> 18
   <212> DNA
   <213> artificial sequence
<220>
   <223> Probe for L1077P
<400> 58
   gttgtggaac ggagtttc 18
<210> 59
   <211> 21
   <212> DNA
   <213> artificial sequence
<220>
   <223> Probe for dele22,23
<400> 59
   cctggcctca agttctgaaa g 21
<210> 60
   <211> 18
   <212> DNA
   <213> artificial sequence
<220>
   <223> Probe for dele2
<400> 60
   ccagaacagt gtcaaaat 18
<210> 61
   <211> 22
   <212> DNA
   <213> artificial sequence
<220>
   <223> Probe for 1677delTA
<400> 61
   tgatgaatag atacagaagc cc 22
<210> 62
   <211> 17
   <212> DNA
   <213> artificial sequence
<220>
   <223> Probe for M1V
<400> 62
   cctctgcacg gtctctc 17
<210> 63
   <211> 18
   <212> DNA
   <213> artificial sequence
<220>
   <223> Probe for E92X
<400> 63
   ttttgtagta agtcacca 18
<210> 64
   <211> 22
   <212> DNA
   <213> artificial sequence
<220>
   <223> Probe for 1584+18672A>G
<400> 64
   agaaatgtac gtactattca tt 22
<210> 65
   <211> 20
   <212> DNA
   <213> artificial sequence
<220>
   <223> Probe for G178R
<400> 65
   taagtattag acaacttgtt 20
<210> 66
   <211> 20
   <212> DNA
   <213> artificial sequence
<220>
   <223> Probe for E822X
<400> 66
   ttaatttctt aacttatttc 20
<210> 67
   <211> 15
   <212> DNA
   <213> artificial sequence
<220>
   <223> Probe for L206W
<400> 67
   cacttgccaa ggagc 15
<210> 68
   <211> 17
   <212> DNA
   <213> artificial sequence
<220>
   <223> Probe for I336K
<400> 68
   cggaaaaaat tcaccac 17
<210> 69
   <211> 16
   <212> DNA
   <213> artificial sequence
<220>
   <223> Probe for R352Q
<400> 69
   ggtcactcag caattt 16
<210> 70
   <211> 19
   <212> DNA
   <213> artificial sequence
<220>
   <223> Probe for M1101K
<400> 70
   gttccaaaag agaatagaa 19
<210> 71
   <211> 17
   <212> DNA
   <213> artificial sequence
<220>
   <223> Probe for 1609delCA
<400> 71
   ctgttctgtt ttcctgg 17
<210> 72
   <211> 18
   <212> DNA
   <213> artificial sequence
<220>
   <223> Probe for D110E
<400> 72
   ttatccggtt cataggaa 18
<210> 73
   <211> 20
   <212> DNA
   <213> artificial sequence
<220>
   <223> Probe for 1811+1,6kbA>G
<400> 73
   ccttacttac atctcaagta 20
<210> 74
   <211> 20
   <212> DNA
   <213> artificial sequence
<220>
   <223> Probe for 2184insA
<400> 74
   aaagattgtt ttttttgttt 20
<210> 75
   <211> 17
   <212> DNA
   <213> artificial sequence
<220>
   <223> Probe for 712-1G>T
<400> 75
   attttccatg gacttgc 17
<210> 76
   <211> 20
   <212> DNA
   <213> artificial sequence
<220>
   <223> Probe for Q890X
<400> 76
   cctttgtctt aaagaggagt 20
<210> 77
   <211> 19
   <212> DNA
   <213> artificial sequence
<220>
   <223> Probe for Y1092X
<400> 77
   ttgacagtta caagaacca 19
<210> 78
   <211> 16
   <212> DNA
   <213> artificial sequence
<220>
   <223> Probe for L346P
<400> 78
   cattgttccg cgcatg 16
<210> 79
   <211> 17
   <212> DNA
   <213> artificial sequence
<220>
   <223> Probe for 4374+1G>A
<400> 79
   caatttttga tgagtct 17
<210> 80
   <211> 23
   <212> DNA
   <213> artificial sequence
<220>
   <223> Primer I12i.3-Bio
<400> 80
   cattctgcca taccaacaat ggt 23
<210> 81
   <211> 20
   <212> DNA
   <213> artificial sequence
<220>
   <223> Primer I12i.5- Bio
<400> 81
   gtgaatcgat gtggtgacca 20
<210> 82
   <211> 20
   <212> DNA
   <213> artificial sequence
<220>
   <223> Primer I16i.3-Bio
<400> 82
   gcaatagaca ggacttcaac 20
<210> 83
   <211> 20
   <212> DNA
   <213> artificial sequence
<220>
   <223> Primer I16i.5-Bio
<400> 83
   cagagaaatt ggtcgttact 20
<210> 84
   <211> 20
   <212> DNA
   <213> artificial sequence
<220>
   <223> Primer Ex10i.3-Bio
<400> 84
   cattcacagt agcttaccca 20
<210> 85
   <211> 20
   <212> DNA
   <213> artificial sequence
<220>
   <223> Primer EX10i.5-Bio
<400> 85
   gcagagtacc tgaaacagga 20
<210> 86
   <211> 24
   <212> DNA
   <213> artificial sequence
<220>
   <223> Primer EX11i.3-Bio
<400> 86
   gcacagattc tgagtaacca taat 24
<210> 87
   <211> 24
   <212> DNA
   <213> artificial sequence
<220>
   <223> Primer Ex11i.5-Bio
<400> 87
   caactgtggt taaagcaata gtgt 24
<210> 88
   <211> 21
   <212> DNA
   <213> artificial sequence
<220>
   <223> Primer Ex20i.3-Bio
<400> 88
   ctatgagaaa actgcactgg a 21
<210> 89
   <211> 21
   <212> DNA
   <213> artificial sequence
<220>
   <223> Primer Ex20i.5- Bio
<400> 89
   ggtcaggatt gaaagtgtgc a 21
<210> 90
   <211> 21
   <212> DNA
   <213> artificial sequence
<220>
   <223> Primer Ex21i.3-Bio
<400> 90
   atttcagtta gcagccttac c 21
<210> 91
   <211> 21
   <212> DNA
   <213> artificial sequence
<220>
   <223> Primer Ex21i.5-Bio
<400> 91
   aaaatgttca caagggactc c 21
<210> 92
   <211> 21
   <212> DNA
   <213> artificial sequence
<220>
   <223> Primer I5i.3- Bio
<400> 92
   ctgtcaagcc gtgttctaga t 21
<210> 93
   <211> 21
   <212> DNA
   <213> artificial sequence
<220>
   <223> Primer I5.5-Bio
<400> 93
   cgcctttcca gttgtataat t 21
<210> 94
   <211> 20
   <212> DNA
   <213> artificial sequence
<220>
   <223> Primer Ex4i.3-Bio
<400> 94
   ttgtaccagc tcactaccta 20
<210> 95
   <211> 18
   <212> DNA
   <213> artificial sequence
<220>
   <223> Primer Ex4i.5- Bio
<400> 95
   gaagtcacca aagcagta 18
<210> 96
   <211> 20
   <212> DNA
   <213> artificial sequence
<220>
   <223> Primer Ex2i3-bio
<400> 96
   agccaccata cttggctcct 20
<210> 97
   <211> 20
   <212> DNA
   <213> artificial sequence
<220>
   <223> Primer Ex2i5-Bio
<400> 97
   tccatatgcc agaaaagttg 20
<210> 98
   <211> 23
   <212> DNA
   <213> artificial sequence
<220>
   <223> Primer Ex3i3-Bio
<400> 98
   ctattcacca gatttcgtag tct 23
<210> 99
   <211> 23
   <212> DNA
   <213> artificial sequence
<220>
   <223> Primer Ex3i5-Bio
<400> 99
   cttgggttaa tctccttgga tat 23
<210> 100
   <211> 28
   <212> DNA
   <213> artificial sequence
<220>
   <223> Primer Ex7i3-Bio
<400> 100
   ggtacattac ctgtattttg tttattgc 28
<210> 101
   <211> 28
   <212> DNA
   <213> artificial sequence
<220>
   <223> Primer Ex7i5-Bio
<400> 101
   catgctcaga tcttccattc caagatcc 28
<210> 102
   <211> 30
   <212> DNA
   <213> artificial sequence
<220>
   <223> Primer I8/Ex9i3-Bio
<400> 102
   ggaagaaaaa gccactgaaa ataatatgag 30
<210> 103
   <211> 30
   <212> DNA
   <213> artificial sequence
<220>
   <223> Primer I8/Ex9i5-Bio
<400> 103
   taattgtaca taaaacaagc atctattgaa 30
<210> 104
   <211> 28
   <212> DNA
   <213> artificial sequence
<220>
   <223> Primer Ex13i3-Bio
<400> 104
   cgtatagagt tgattggatt gagaatag 28
<210> 105
   <211> 28
   <212> DNA
   <213> artificial sequence
<220>
   <223> Primer Ex13i5-Bio
<400> 105
   ctttcgacca atttagtgca gaaagaag 28
<210> 106
   <211> 22
   <212> DNA
   <213> artificial sequence
<220>
   <223> Primer Ex14bi3-Bio
<400> 106
   tgggagaaat gaaacaaagt gg 22
<210> 107
   <211> 22
   <212> DNA
   <213> artificial sequence
<220>
   <223> Primer Ex14bi5-Bio
<400> 107
   gaacacctag tacagctgct gg 22
<210> 108
   <211> 20
   <212> DNA
   <213> artificial sequence
<220>
   <223> Primer Ex17Ai3-Bio
<400> 108
   caccaactgt ggtaagattc 20
<210> 109
   <211> 20
   <212> DNA
   <213> artificial sequence
<220>
   <223> Primer Ex17Ai5-Bio
<400> 109
   acacactttg tccactttgc 20
<210> 110
   <211> 20
   <212> DNA
   <213> artificial sequence
<220>
   <223> Primer I17A/Ex17Bi3-Bio
<400> 110
   tgagttcata gtacctgttg 20
<210> 111
   <211> 20
   <212> DNA
   <213> artificial sequence
<220>
   <223> Primer I17A/Ex17bi5-Bio
<400> 111
   caaagaatgg caccagtgtg 20
<210> 112
   <211> 20
   <212> DNA
   <213> artificial sequence
<220>
   <223> Primer Ex19i3-Bio
<400> 112
   cagtctaaca aagcaagcag 20
<210> 113
   <211> 20
   <212> DNA
   <213> artificial sequence
<220>
   <223> Primer Ex19i5-bio
<400> 113
   gcccgacaaa taaccaagtg 20
<210> 114
   <211> 20
   <212> DNA
   <213> artificial sequence
<220>
   <223> Primer I19i3-Bio
<400> 114
   ggtataagca gcatattctc 20
<210> 115
   <211> 20
   <212> DNA
   <213> artificial sequence
<220>
   <223> Primer I19i5-Bio
<400> 115
   ggcttctcag tgatctgttg 20
<210> 116
   <211> 23
   <212> DNA
   <213> artificial sequence
<220>
   <223> Primer I1/I3i3-Bio
<400> 116
   gcatacaaaa aatggaagaa agc 23
<210> 117
   <211> 26
   <212> DNA
   <213> artificial sequence
<220>
   <223> Primer I1/I3i5-Bio
<400> 117
   ctttaatggt gtttacctac ctagag 26
<210> 118
   <211> 28
   <212> DNA
   <213> artificial sequence
<220>
   <223> Primer Ex6ai3-Bio
<400> 118
   gttttactaa agtgggcttt ttgaaaac 28
<210> 119
   <211> 27
   <212> DNA
   <213> artificial sequence
<220>
   <223> Primer Ex6ai5-Bio
<400> 119
   tagtttctag gggtggaaga tacaatg 27
<210> 120
   <211> 20
   <212> DNA
   <213> artificial sequence
<220>
   <223> Primer Ex6bi3-Bio
<400> 120
   aggcatttac caaacagtag 20
<210> 121
   <211> 21
   <212> DNA
   <213> artificial sequence
<220>
   <223> Primer Ex6bi5-Bio
<400> 121
   gcccatgaaa gtgaatttgt g 21
<210> 122
   <211> 28
   <212> DNA
   <213> artificial sequence
<220>
   <223> Primer Ex8i3-Bio
<400> 122
   cttaatctag aaaataagtg agtgatcc 28
<210> 123
   <211> 28
   <212> DNA
   <213> artificial sequence
<220>
   <223> Primer Ex8i5-Bio
<400> 123
   aatagtacaa ggaagaatca gttgtatg 28
<210> 124
   <211> 28
   <212> DNA
   <213> artificial sequence
<220>
   <223> Primer Ex18i3-Bio
<400> 124
   gaagagataa ggatttaatg acagatac 28
<210> 125
   <211> 27
   <212> DNA
   <213> artificial sequence
<220>
   <223> Primer Ex18i5-Bio
<400> 125
   aagtgtgaat aaagtcgttc acagaag 27
<210> 126
   <211> 20
   <212> DNA
   <213> artificial sequence
<220>
   <223> Primer Ex22i3-Bio
<400> 126
   aaatgagcac tgggttcatc 20
<210> 127
   <211> 20
   <212> DNA
   <213> artificial sequence
<220>
   <223> Primer Ex22i5-Bio
<400> 127
   attcaaatgg tggcaggtag 20
<210> 128
   <211> 27
   <212> DNA
   <213> artificial sequence
<220>
   <223> Primer Ex24i3-Bio
<400> 128
   acttaattca tccttgtttt ctgaggc 27
<210> 129
   <211> 28
   <212> DNA
   <213> artificial sequence
<220>
   <223> Primer Ex24i5-Bio
<400> 129
   gacattgcat tctttgactt ttattttc 28
<210> 130
   <211> 20
   <212> DNA
   <213> artificial sequence
<220>
   <223> Primer del Ex1.5-Bio
<400> 130
   agtaggtctt tggcattagg 20
<210> 131
   <211> 20
   <212> DNA
   <213> artificial sequence
<220>
   <223> Primer del Ex1.3-Bio
<400> 131
   cccagggttt ccatattctc 20
<210> 132
   <211> 22
   <212> DNA
   <213> artificial sequence
<220>
   <223> Primer Ex1/In1.3-Bio
<400> 132
   caagtgcata gtagcgtact tg 22
<210> 133
   <211> 23
   <212> DNA
   <213> artificial sequence
<220>
   <223> Primer de1 Ex2.5-Bio
<400> 133
   ttaaccttag agccctaatt cag 23
<210> 134
   <211> 22
   <212> DNA
   <213> artificial sequence
<220>
   <223> Primer del Ex2.3-Bio
<400> 134
   agccatttca cattccatca tg 22
<210> 135
   <211> 23
   <212> DNA
   <213> artificial sequence
<220>
   <223> Primer del Ex2/Ex3.5-Bio
<400> 135
   ccttggtaaa attaagcctc atg 23
<210> 136
   <211> 20
   <212> DNA
   <213> artificial sequence
<220>
   <223> Primer del Ex2ins182.3-Bio
<400> 136
   tgcctgttgt tggtgtaaac 20
<210> 137
   <211> 23
   <212> DNA
   <213> artificial sequence
<220>
   <223> Primer In3.3-Bio
<400> 137
   gcatacaaaa aatggaagaa agc 23
<210> 138
   <211> 20
   <212> DNA
   <213> artificial sequence
<220>
   <223> Primer In1/Ex2.5-Bio
<400> 138
   tccatatgcc agaaaagttg 20
<210> 139
   <211> 20
   <212> DNA
   <213> artificial sequence
<220>
   <223> Primer Ex2/In2.3-Bio
<400> 139
   tagccaccat acttggctcc 20
<210> 140
   <211> 23
   <212> DNA
   <213> artificial sequence
<220>
   <223> Primer del Ex14b/Ex17b.5-Bio
<400> 140
   caaatgtttc caccttgaga aag 23
<210> 141
   <211> 19
   <212> DNA
   <213> artificial sequence
<220>
   <223> Primer del Ex14b/Ex17b.3-Bio
<400> 141
   cccttcaatc acagaattg 19
<210> 142
   <211> 20
   <212> DNA
   <213> artificial sequence
<220>
   <223> Primer del Ex17a/Ex17b/Ex18.5-Bio
<400> 142
   tgcctcatct gaaatgattg 20
<210> 143
   <211> 20
   <212> DNA
   <213> artificial sequence
<220>
   <223> Primer del Ex17a/Ex17b/Ex18.3-Bio
<400> 143
   tttgagggca tagtcctatc 20
<210> 144
   <211> 21
   <212> DNA
   <213> artificial sequence
<220>
   <223> Primer In17a/Ex17b.5-Bio
<400> 144
   aatatttcac aggcaggagt c 21
<210> 145
   <211> 22
   <212> DNA
   <213> artificial sequence
<220>
   <223> Primer Ex17b/In17b.3-Bio
<400> 145
   ttgataacct atagaatgca gc 22
<210> 146
   <211> 20
   <212> DNA
   <213> artificial sequence
<220>
   <223> Primer del Ex22/Ex23.5-Bio
<400> 146
   ggatacctgg cacgtaatag 20
<210> 147
   <211> 20
   <212> DNA
   <213> artificial sequence
<220>
   <223> Primer del Ex22/Ex23.3-Bio
<400> 147
   ccttgtgact aaaccatagg 20
<210> 148
   <211> 19
   <212> DNA
   <213> artificial sequence
<220>
   <223> Primer del Ex22/Ex24.5-Bio
<400> 148
   gattctgctg ccacagatc 19
<210> 149
   <211> 20
   <212> DNA
   <213> artificial sequence
<220>
   <223> Primer del Ex22/Ex24.3-Bio
<400> 149
   cctgtgccta tgaagacaag 20
<210> 150
   <211> 20
   <212> DNA
   <213> artificial sequence
<220>
   <223> Primer Ex22/In22.3-Bio
<400> 150
   aggcataatg attctgttcc 20
<210> 151
   <211> 20
   <212> DNA
   <213> artificial sequence
<220>
   <223> Primer In14b/Ex15.5-Bio
<400> 151
   aactttggct gccaaataac 20
<210> 152
   <211> 21
   <212> DNA
   <213> artificial sequence
<220>
   <223> Primer Ex15/In15.3-Bio
<400> 152
   aaaccacagg ccctattgat g 21
<210> 153
   <211> 28
   <212> DNA
   <213> artificial sequence
<220>
   <223> Primer In11/Ex12.5-Bio
<400> 153
   ttgtaatgca tgtagtgaac tgtttaag 28
<210> 154
   <211> 27
   <212> DNA
   <213> artificial sequence
<220>
   <223> Primer Ex12/In12.3-Bio
<400> 154
   ataccaacaa tggtgaacat atttctc 27
<210> 155
   <211> 30
   <212> DNA
   <213> artificial sequence
<220>
   <223> Primer In9/Ex10.5-Bio
<400> 155
   aatatacact tctgcttagg atgataattg 30
<210> 156
   <211> 28
   <212> DNA
   <213> artificial sequence
<220>
   <223> Primer Ex10/In10.3-Bio
<400> 156
   ttcacagtag cttacccata gaggaaac 28
<210> 157
   <211> 22
   <212> DNA
   <213> artificial sequence
<220>
   <223> Primer CFTR pri In3.5v1-Bio
<400> 157
   gtcttgtgtt gaaattctca gg 22
<210> 158
   <211> 26
   <212> DNA
   <213> artificial sequence
<220>
   <223> Primer CFTR pri In11.5v2-Bio
<400> 158
   gtgtatatag ccaagttatt gtacag 26
<210> 159
   <211> 21
   <212> DNA
   <213> artificial sequence
<220>
   <223> Primer CFTR pri In11.3-Bio
<400> 159
   agcaatagtc tactgttgac c 21
<210> 160
   <211> 21
   <212> DNA
   <213> artificial sequence
<220>
   <223> Primer CFTR pri In12.5-Bio
<400> 160
   gtgtacattt tgatgggatc c 21
<210> 161
   <211> 21
   <212> DNA
   <213> artificial sequence
<220>
   <223> Primer CFTR pri In12.3-Bio
<400> 161
   aaagatgaag acacagttcc c 21
<210> 162
   <211> 22
   <212> DNA
   <213> artificial sequence
<220>
   <223> Primer CFTR pri Ex14.5-Bio
<400> 162
   caggcaaact tgactgaact gg 22
<210> 163
   <211> 26
   <212> DNA
   <213> artificial sequence
<220>
   <223> Primer CFTR pri In14.3-Bio
<400> 163
   tacatgctac atattgcatt ctactc 26
<210> 164
   <211> 25
   <212> DNA
   <213> artificial sequence
<220>
   <223> Primer CFTR pri In25.5-Bio
<400> 164
   tactgttctg tgatattatg tgtgg 25
<210> 165
   <211> 25
   <212> DNA
   <213> artificial sequence
<220>
   <223> Primer CFTR pri In26.3-Bio
<400> 165
   aactatcaca tgtgagattg ttatc 25